# EUROPEAN PATENT SPECIFICATION

(11) **EP 4 017 566 B1**
(45) Date of publication and mention of the grant of the patent: **13.05.2026**
(21) Application number: 20857253.7
(22) Date of filing: 21.08.2020
(51) Int. Cl.: A61M 16/16, A61M 16/14

(54) **LIQUID CHAMBER FOR A BREATHING ASSISTANCE APPARATUS**
FLÜSSIGKEITSKAMMER FÜR EIN BEATMUNGSGERÄT
CHAMBRE DE LIQUIDE POUR UN APPAREIL D'ASSISTANCE RESPIRATOIRE

(30) Priority: 23.08.2019 US 201962890846 P
(43) Date of publication of application: 29.06.2022
(60) Divisional application: 26170003.3
(73) Proprietor: Fisher & Paykel Healthcare Limited, East Tamaki Auckland 2013 (NZ)
(72) Inventor: KRAMER, Martin Paul Friedrich, Auckland, 2013 (NZ); GARCIA, Enrico Alvarez, Auckland, 2013 (NZ)
(74) Representative: Forresters IP LLP
(86) International application number: PCT/IB2020/057846
(87) International publication number: WO 2021/038401

(56) References cited:
- EP-A2- 2 319 568
- WO-A1-2004/026382
- WO-A1-2013/147623
- WO-A1-2013/174129
- WO-A1-2018/199774
- WO-A2-2007/038152
- DE-A1- 2 447 113
- US-A- 3 659 604
- US-A- 4 652 408
- US-A- 5 738 808
- US-A- 5 943 473

## Description

### TECHNICAL FIELD

The present invention relates to a liquid chamber for a breathing assistance apparatus.

### BACKGROUND ART

Breathing assistance apparatuses are used in various environments such as hospital, medical facility, residential care, or home environments to deliver a flow of gas to users or patients. The breathing assistance apparatuses come in various forms, such as a standalone humidifier apparatus, a continuous positive airway pressure (CPAP) apparatus, or a high flow apparatus.

A standalone humidifier apparatus can deliver heated and humidified gases for various medical procedures, including respiratory therapy, laparoscopy, and the like. These apparatuses can be configured to control temperature and/or humidity. The apparatuses can also include medical circuits comprising various components that can be used to transport heated and/or humidified gases to and from patients. For example, in some breathing circuits, gases inhaled by a patient are delivered from a heater-humidifier through an inspiratory tube or conduit. As another example, tubes can deliver humidified gas (commonly CO₂) into the abdominal cavity in insufflation circuits. This can help prevent desiccation or 'drying out' of the patient's internal organs, and can decrease the amount of time needed for recovery from surgery. Heater wires may extend inside of at least a portion of the tubing forming the circuit to prevent or at least reduce the likelihood of the formation of significant condensation.

A standalone humidifier apparatus would typically include a heater base and a humidifier liquid chamber. The heater base can comprise a heater plate. The liquid chamber can be configured to hold a volume of a liquid, such as water. The heater plate can be configured to heat the volume of liquid held within the liquid chamber to produce vapour.

The liquid chamber is removable from the heater base to allow the liquid chamber to be more readily sterilized or disposed, or to re-fill the chamber with liquid. The body of the liquid chamber can be formed from a non-conductive glass or plastics material but the liquid chamber can also include conductive components. For instance, the liquid chamber can include a highly heat-conductive base (for example, an aluminum base) contacting or associated with the heater plate on the heater base.

The heater base can also include electronic controls such as a master controller. In response to user-set humidity or temperature values input via a user interface and other inputs, the master controller determines when (or to what level) to energize the heater plate to heat the liquid within the liquid chamber.

The standalone humidifier apparatus can include a gases supply to deliver gases to the liquid chamber. In some configurations, the gases supply can comprise a ventilator, blower, or any other suitable source of pressurized gases suitable for breathing or use in medical procedures.

A standalone humidifier apparatus can be used with breathing therapies, positive pressure apparatus, noninvasive ventilation, surgical procedures including but not limited to laparoscopy, and the like. Desirably, the humidifier apparatus can be adapted to supply humidity or vapour to a supply of gases. The humidifier apparatus can be used with continuous, variable, or bi-level PAP systems or other form of respiratory therapy. In some configurations, the humidifier apparatus can be integrated into a system that delivers any such types of therapy.

An exemplary standalone humidifier apparatus is described in WO 2015/038013.

A CPAP apparatus is a gases supply and optionally gases humidification apparatus. The apparatus is operable to provide respiratory assistance to patients or users who require a supply of gas (humidified or otherwise) at positive pressure for the treatment of diseases such as Obstructive Sleep Apnea (OSA), snoring, or Chronic Obstructive Pulmonary Disease (COPD) and the like. A CPAP apparatus would typically include a humidifier liquid chamber, so as to form a combined assisted breathing unit and humidifier.

CPAP apparatuses, when used with a humidifier, typically have a structure where gases at a required pressure are delivered from an assisted breathing unit or blower unit to a liquid chamber downstream from the blower. As the gases pass through the liquid chamber, they become saturated with liquid vapour (e.g. water vapour). A flexible tubular gases conduit delivers the gases to a user or patient downstream from the humidifier chamber.

An exemplary CPAP apparatus is described in WO 2011/056080. US5943473A discloses a heated cartridge humidifier for delivering humidified respiratory gases to patients. A humidifier housing includes a gas and water supply and a gas outlet. A humidifier base plate engages a heating element. The base plate includes a central heat conductive portion retained in an outer insulating portion which prevents the lateral dissipation of heat received from the heating element. The baseplate enhances the heat transfer to the water, and therefore, enhances the humidity of the gases. WO2007038152A2 discloses a pressure support system that comprises a patient circuit, a docking assembly, and a tank. The patient circuit delivers a pressurized flow of breathable gas to a patient. The docking assembly has an inlet and an outlet that is adapted to receive the pressurized flow of breathable gas, and is also adapted to be connected with the patient circuit. The tank is constructed and arranged to be removably connected with the docking assembly, and enables the pressurized flow of breathable gas to pass therethrough. The tank is also adapted to contain a liquid such that a humidity level of the pressurized flow of breathable gas is elevated as the pressurized flow of breathable gas passes therethrough.
WO 2004/026382 A1 discloses an apparatus for delivering humidified gases which has a connection manifold adapted to connect with inlet and outlet ports of a slide-on water chamber in a single slide on motion. Connection of the gases inlet and gases outlet ports as well as any additional electrical and/or pneumatic connections are all made in the same slide on motion. The water chamber may include inwardly extending elongate extension tubes and at least one of the extension tubes may also have an air bleed aperture to aid filling of the chamber.

A high flow apparatus may be used to deliver a high gas flow or high flow therapy to a patient to assist with breathing and/or treat breathing disorders including chronic obstructive pulmonary disease (COPD). A high flow apparatus includes a gases supply and typically includes a humidification apparatus.

The breathing assistance apparatuses typically have one or more accessories such as a breathing conduit and a patient interface such as a cannula or mask for delivering gases to a patient. The conduit enables gases to be delivered from the housing of the breathing assistance apparatus to the patient. For example, the apparatus may be placed on a floor or other support surface, and the patient may be in a bed. The breathing assistance apparatus may have a recess for receipt of a humidifier liquid chamber. The liquid chamber will receive liquid from, for example, a flexible liquid bag that delivers liquid to a humidifier liquid chamber via one or more tubes. Alternatively, the liquid chamber can be removed and refilled as required. The recess will contain a heater plate to heat the liquid chamber, to humidify gases passing through the liquid chamber. The humidified gases are then delivered to the patient.

### SUMMARY

In accordance with certain features, aspects and advantages of the invention, a humidifier liquid chamber or a humidifier liquid chamber manifold is provided in accordance with appended claim 1. Preferred embodiments are matter of the dependent claims.
According to the invention, the humidifier liquid chamber or humidifier liquid chamber manifold comprises:
first and second base unit connection ports for connecting to a breathing assistance apparatus base unit, the base unit connection ports defining respective axes;
wherein the base unit connection ports are substantially parallel to each other and are separated by a port separation distance between the axes;
wherein at least one of the base unit connection ports has a sealing depth defined by a portion of the port that is configured to overlap with a complementary chamber connection port of the base unit, wherein a ratio of the sealing depth to port separation distance is more than about 0.25 and up to about 0.7.

In some configurations, the ratio of the sealing depth to port separation distance is between about 0.3 and about 0.7.

In some configurations, the ratio of the sealing depth to port separation distance is about 0.475.

In some configurations, the sealing depth is more than about 10 mm.

In some configurations, the sealing depth is more than about 10 mm and up to about 16 mm.

In some configurations, the sealing depth is more than about 12 mm.

In some configurations, the sealing depth is more than about 12 mm and up to about 16 mm.

In some configurations, the at least one of the base unit connection ports is configured to seal with at least two seals on the complementary chamber connection port.

In some configurations, the base unit connection ports each have an exterior face, and wherein the exterior faces are substantially planar and are substantially perpendicular to the axes.

In some configurations, the humidifier liquid chamber manifold comprises first and second manifold-to-chamber connection ports for connecting to a humidifier liquid chamber, wherein the first manifold-to-chamber connection port is in fluid communication with the first base unit connection port, and wherein the second manifold-to-chamber connection port is in fluid communication with the second base unit connection port.

In some configurations of the humidifier liquid chamber manifold, a first conduit extends between and fluidly connects the first manifold-to-chamber connection port and the first base unit connection port, and a second conduit extends between and fluidly connects the second manifold-to-chamber connection port and the second base unit connection port.

According to the invention, the axes of the first and second base unit connection ports are configured to be in a substantially horizontal orientation when the humidifier liquid chamber manifold is in use.

In some configurations of the humidifier liquid chamber manifold, axes of the first and second manifold-to-chamber connection ports are configured to be in a substantially vertical orientation when the humidifier liquid chamber manifold is in use.

In some configurations of the humidifier liquid chamber manifold, the humidifier liquid chamber manifold comprises an auxiliary port that is in communication with an interior of the humidifier liquid chamber manifold.

In some configurations of the humidifier liquid chamber manifold, the humidifier liquid chamber manifold comprises a conduit clip to engage with a liquid conduit for delivering liquid to a humidifier liquid chamber.

In some configurations, the humidifier liquid chamber manifold is integrally formed as part of a humidifier liquid chamber.

In some configurations, the port separation distance is between about 35 mm and about 45 mm.

In some configurations, the port separation distance is between about 38 mm and about 42 mm.

In some configurations, the port separation distance is about 40 mm +/- 0.6mm.

In some configurations, the humidifier liquid chamber comprises flow tubes extending into the humidifier liquid chamber from the first and second base unit connection ports.

In some configurations of the humidifier liquid chamber, each flow tube comprises an opening at or adjacent an end of the flow tube that is distal from the respective base unit connection port, wherein the opening is configured to direct flow and is spaced from a wall of the humidifier liquid chamber.

In some configurations of the humidifier liquid chamber, the opening in each flow tube faces a roof of the humidifier liquid chamber.

In some configurations of the humidifier liquid chamber, the humidifier liquid chamber comprises a baffle in the interior of the humidifier liquid chamber and between the openings of the flow tubes.

In some configurations of the humidifier liquid chamber, the baffle extends downward from a roof of the humidifier liquid chamber and terminates above a maximum liquid fill level of the humidifier liquid chamber.

In some configurations of the humidifier liquid chamber, a distance between a bottom edge of the baffle and the maximum liquid fill level is between about 5 mm and about 15 mm.

In some configurations of the humidifier liquid chamber, the baffle extends downward from a roof of the humidifier liquid chamber and terminates at or below a maximum liquid fill level of the humidifier liquid chamber.

In some configurations of the humidifier liquid chamber, a roof of the humidifier liquid chamber is sloped so as to be substantially non-horizontal in use.

In some configurations of the humidifier liquid chamber, at least one of the roof, the baffle, and wall(s) of the humidifier liquid chamber are configured to direct gases exiting the opening of the flow tube associated with the first base unit connection port toward and over a surface of liquid in the humidifier liquid chamber.

In some configurations of the humidifier liquid chamber, the humidifier liquid chamber comprises substantially no deadspace in the humidifier liquid chamber in use.

In some configurations of the humidifier liquid chamber, each flow tube comprises a liquid drainage aperture at or adjacent an end of the flow tube that is distal from the respective base unit connection port.

In some configurations of the humidifier liquid chamber, each flow tube comprises a gas bleed aperture in an upper surface of the flow tube.

In some configurations of the humidifier liquid chamber, the humidifier liquid chamber comprises a step in the humidifier liquid chamber.

In some configurations of the humidifier liquid chamber, a base of the step is located at least partly below the first and second base unit connection ports.

In some configurations of the humidifier liquid chamber, the baffle extends from a rear of the humidifier liquid chamber to a substantially vertical face of the step.

In some configurations of the humidifier liquid chamber, the baffle extends from a rear of the humidifier liquid chamber towards a substantially vertical face of the step, and terminates proximal to a central axis of the humidifier liquid chamber.

In some configurations of the humidifier liquid chamber, the humidifier liquid chamber has a substantially cylindrical shape.

In some configurations of the humidifier liquid chamber, the humidifier liquid chamber tapers towards a centre of the humidifier liquid chamber from a base of the humidifier liquid chamber to a top of the humidifier liquid chamber.

In some configurations of the humidifier liquid chamber, the humidifier liquid chamber comprises a heat conductive base.

In some configurations of the humidifier liquid chamber, the base of the humidifier liquid chamber is removable from a body of the humidifier liquid chamber.

In some configurations of the humidifier liquid chamber, the humidifier liquid chamber comprises one or more base removal features to assist with removing the base of the humidifier liquid chamber from the body of the humidifier liquid chamber.

In some configurations of the humidifier liquid chamber, the one or more base removal features is/are located between a lower flange of the body of the humidifier liquid chamber and an outwardly extending flange of the base of the humidifier liquid chamber.

In some configurations of the humidifier liquid chamber, the one or more base removal features comprises a slot.

In some configurations of the humidifier liquid chamber, the humidifier liquid chamber comprises a shoulder between the first and second base unit connection ports.

In some configurations of the humidifier liquid chamber, the shoulder extends beyond exterior faces of the first and second base unit connection ports.

In some configurations of the humidifier liquid chamber, one of the base unit connection ports is an inlet port and the other of the base unit connection ports is an outlet port.

Additionally, in accordance with certain features, aspects and advantages of at least one of the embodiments disclosed herein, a humidifier liquid chamber or a humidifier liquid chamber manifold is disclosed, the humidifier liquid chamber or humidifier liquid chamber manifold comprising:
first and second base unit connection ports for connecting to a breathing assistance apparatus base unit;
wherein the base unit connection ports are substantially parallel to each other;
wherein each of the base unit connection ports comprises at least two sealing portions configured to form at least two seals with a portion of the breathing assistance apparatus base unit when connected to the breathing assistance apparatus base unit.

In some configurations, the two sealing portions are separated by a distance of about 5 mm to about 7 mm.

In some configurations, the two sealing portions are separated by a distance of about 5.5 mm to about 6.5 mm.

In some configurations, each base unit connection port comprises an internal taper such that the base unit connection port narrows from a terminal end of the base unit connection port towards an opposite end of the base unit connection port.

In some configurations, an inner diameter of a first one of the sealing portions proximal to the terminal end of the base unit connection port is about 0 mm to about 0.6 mm greater than an inner diameter of a second one of the sealing portions distal from the terminal end of the base unit connection port.

In some configurations, the inner diameter of the first one of the sealing portions is less than about 25.6 mm.

In some configurations, the inner diameter of the second one of the sealing portions is less than about 25.4 mm.

In some configurations of the humidifier liquid chamber, each base unit connection port comprises a central axis that is at least about 85 mm above a lower surface of a body of the humidifier liquid chamber.

In some configurations of the humidifier liquid chamber, the central axes of the base unit connection ports are at least about 88 mm above the lower surface of the body of the humidifier liquid chamber.

In some configurations of the humidifier liquid chamber, the lower surface is defined by an upper edge of a lower flange of the body of the humidifier liquid chamber.

In some configurations of the humidifier liquid chamber, the lower flange is configured to engage with one or more retaining features in a breathing assistance apparatus base unit to assist with retaining the humidifier liquid chamber in engagement with the breathing assistance apparatus base unit.

In some configurations of the humidifier liquid chamber or humidifier liquid chamber manifold, each base unit connection port is cylindrical.

In some configurations of the humidifier liquid chamber or humidifier liquid chamber manifold, each base unit connection port is configured to receive a complementary port of a breathing assistance apparatus base unit.

In some configurations of the humidifier liquid chamber, the humidifier liquid chamber comprises flow tubes extending into the humidifier liquid chamber from the first and second base unit connection ports.

In some configurations of the humidifier liquid chamber, the liquid chamber comprises a baffle in the interior of the humidifier liquid chamber and between openings of the flow tubes.

In some configurations of the humidifier liquid chamber, the baffle extends downward from a roof of the humidifier liquid chamber and terminates above a maximum liquid fill level of the humidifier liquid chamber.

In some configurations of the humidifier liquid chamber, the baffle extends downward from a roof of the humidifier liquid chamber and terminates at or below a maximum liquid fill level of the humidifier liquid chamber.

In some configurations of the humidifier liquid chamber, a roof of the humidifier liquid chamber is sloped so as to be substantially non-horizontal in use.

In some configurations of the humidifier liquid chamber, at least one of the roof, the baffle, and wall(s) of the humidifier liquid chamber are configured to direct gases exiting the opening of the flow tube associated with the first base unit connection port toward and over a surface of liquid in the humidifier liquid chamber.

In some configurations of the humidifier liquid chamber, the humidifier liquid chamber comprises a step in the humidifier liquid chamber.

In some configurations of the humidifier liquid chamber, a base of the step is located at least partly below the first and second base unit connection ports.

In some configurations of the humidifier liquid chamber, the baffle extends from a rear of the humidifier liquid chamber towards a substantially vertical face of the step, and terminates proximal to a central axis of the humidifier liquid chamber.

In some configurations of the humidifier liquid chamber, a distance between a forward edge of the baffle and the substantially vertical face of the step is at least about 20 mm.

In some configurations of the humidifier liquid chamber or humidifier liquid chamber manifold, the first and second base unit connection ports define respective axes, and the first and second base unit connection ports are separated by a port separation distance between the axes, and wherein the port separation distance is between about 35 mm and about 45 mm.

In some configurations of the humidifier liquid chamber or humidifier liquid chamber manifold, the port separation distance is between about 38 mm and about 42 mm.

In some configurations of the humidifier liquid chamber or humidifier liquid chamber manifold, the port separation distance is about 40 mm +/- 0.6mm

Additionally, in accordance with certain features, aspects and advantages of at least one of the embodiments disclosed herein, a humidifier liquid chamber is disclosed, the humidifier liquid chamber comprising:
an inlet port and an outlet port, the inlet port and the outlet port defining respective axes;
wherein the inlet port and the outlet port are substantially parallel to each other and are configured to be in a substantially horizontal orientation when the humidifier liquid chamber is in use, and wherein the inlet port and the outlet port are separated by a port separation distance between the axes;
wherein at least one of the inlet port and the outlet port has a sealing depth defined by a portion of the port that is configured to overlap with a complementary chamber connection port of a breathing assistance apparatus base unit, wherein a ratio of the sealing depth to port separation distance is more than about 0.25 and up to about 0.7.

In some configurations, the ratio of the sealing depth to port separation distance is between about 0.3 and about 0.7.

In some configurations, the ratio of the sealing depth to port separation distance is about 0.475.

In some configurations, the sealing depth is more than about 10 mm.

In some configurations, the sealing depth is more than about 10 mm and up to about 16 mm.

In some configurations, the sealing depth is more than about 12 mm.

In some configurations, the sealing depth is more than about 12 mm and up to about 16 mm.

In some configurations, the at least one of the inlet port and the outlet port is configured to seal with at least two seals on the complementary chamber connection port.

In some configurations, the port separation distance is between about 35 mm and about 45 mm.

In some configurations, the port separation distance is between about 38 mm and about 42 mm.

In some configurations, the port separation distance is about 40 mm +/- 0.6mm.

In some configurations, the humidifier liquid chamber comprises flow tubes extending into the humidifier liquid chamber from the inlet port and the outlet port.

In some configurations of the humidifier liquid chamber, each flow tube comprises an opening at or adjacent an end of the flow tube that is distal from the respective port, wherein the opening is configured to direct flow and is spaced from a wall of the humidifier liquid chamber.

In some configurations, the opening in each flow tube faces a roof of the humidifier liquid chamber.

In some configurations, the humidifier liquid chamber comprises a baffle in the interior of the humidifier liquid chamber and between the openings of the flow tubes.

In some configurations, the baffle extends downward from a roof of the humidifier liquid chamber and terminates above a maximum liquid fill level of the humidifier liquid chamber.

In some configurations, a distance between a bottom edge of the baffle and the maximum liquid fill level is between about 5 mm and about 15 mm.

In some configurations, the baffle extends downward from a roof of the humidifier liquid chamber and terminates at or below an intended maximum liquid fill level of the humidifier liquid chamber.

In some configurations, a roof of the humidifier liquid chamber is sloped so as to be substantially non-horizontal in use.

In some configurations, at least one of the roof, the baffle, and wall(s) of the humidifier liquid chamber are configured to direct gases exiting the opening of the flow tube associated with the inlet port toward and over a surface of liquid in the humidifier liquid chamber.

In some configurations, the humidifier liquid chamber comprises substantially no deadspace in the humidifier liquid chamber in use.

In some configurations, each flow tube comprises a liquid drainage aperture at or adjacent an end of the flow tube that is distal from the respective port of the humidifier liquid chamber.

In some configurations, each flow tube comprises an air bleed aperture in an upper surface of the flow tube.

In some configurations, the humidifier liquid chamber comprises a step in the humidifier liquid chamber.

In some configurations, a base of the step is located at least partly below the inlet port and the outlet port.

In some configurations, the baffle extends from a rear of the humidifier liquid chamber to a substantially vertical face of the step.

In some configurations, the baffle extends from a rear of the humidifier liquid chamber towards a substantially vertical face of the step, and terminates proximal to a central axis of the humidifier liquid chamber.

In some configurations, the humidifier liquid chamber has a substantially cylindrical shape.

In some configurations, the humidifier liquid chamber tapers towards a centre of the humidifier liquid chamber from a base of the humidifier liquid chamber to a top of the humidifier liquid chamber.

In some configurations, the humidifier liquid chamber comprises a heat conductive base.

In some configurations of the humidifier liquid chamber, the base of the humidifier liquid chamber is removable from a body of the humidifier liquid chamber.

In some configurations of the humidifier liquid chamber, the humidifier liquid chamber comprises one or more base removal features to assist with removing the base of the humidifier liquid chamber from the body of the humidifier liquid chamber.

In some configurations of the humidifier liquid chamber, the one or more base removal features is/are located between a lower flange of the body of the humidifier liquid chamber and an outwardly extending flange of the base of the humidifier liquid chamber.

In some configurations of the humidifier liquid chamber, the one or more base removal features comprises a slot.

In some configurations, the humidifier liquid chamber comprises a shoulder between the inlet port and the outlet port.

In some configurations, the shoulder extends beyond exterior faces of the inlet port and the outlet port.

Additionally, in accordance with certain features, aspects and advantages of at least one of the embodiments disclosed herein, a humidifier liquid chamber manifold is disclosed, the humidifier liquid chamber manifold comprising:
a base unit connection portion comprising:
   first and second base unit connection ports for connecting to a breathing assistance apparatus base unit, the base unit connection ports defining respective axes;
   wherein the base unit connection ports are substantially parallel to each other and are separated by a port separation distance between the axes;
   wherein at least one of the base unit connection ports has a sealing depth defined by a portion of the port that is configured to overlap with a complementary connection port of the base unit, wherein the ratio of the sealing depth to the port separation distance is more than about 0.25; and
a humidifier liquid chamber connection portion comprising:
   first and second manifold-to-chamber connection ports for connecting to a humidifier liquid chamber, wherein the first manifold-to-chamber connection port is in fluid communication with the first base unit connection port, and wherein the second manifold-to-chamber connection port is in fluid communication with the second base unit connection port.

In some configurations, the ratio of the sealing depth to the port separation distance is more than about 0.25 and up to about 0.7.

In some configurations, the ratio of the sealing depth to the port separation distance is between about 0.3 and about 0.7.

In some configurations, the ratio of the sealing depth to the port separation distance is about 0.475.

In some configurations, the sealing depth is more than about 10 mm.

In some configurations, the sealing depth is more than about 10 mm and up to about 16mm.

In some configurations, the sealing depth is more than about 12 mm.

In some configurations, the sealing depth is more than about 12 mm and up to about 16 mm.

In some configurations, the at least one of the base unit connection ports is configured to seal with at least two seals on the complementary connection port of the base unit.

In some configurations, the port separation distance is between about 35 mm and about 45 mm.

In some configurations, the port separation distance is between about 38 mm and about 42 mm.

In some configurations, a first conduit extends between and fluidly connects the first manifold-to-chamber connection port and the first base unit connection port, and wherein a second conduit extends between and fluidly connects the second manifold-to-chamber connection port and the second base unit connection port.

In some configurations, the axes of the first and second base unit connection ports are configured to be in a substantially horizontal orientation when the humidifier liquid chamber manifold is in use.

In some configurations, axes of the first and second manifold-to-chamber connection ports are configured to be in a substantially vertical orientation when the humidifier liquid chamber manifold is in use.

In some configurations, the humidifier liquid chamber manifold comprises an auxiliary port that is in communication with an interior of the humidifier liquid chamber manifold.

In some configurations, the humidifier liquid chamber manifold comprises a conduit clip to engage with a liquid conduit for delivering liquid to a humidifier liquid chamber.

Additionally, in accordance with certain features, aspects and advantages of at least one of the embodiments disclosed herein, a humidifier liquid chamber is disclosed, the humidifier liquid chamber comprising:
a base, a top, and one or more side walls;
an inlet port and an outlet port, the inlet port and the outlet port defining axes;
at least two grips in the side wall(s), wherein the grips are located at an angle relative to a plane that extends through a centre of the humidifier liquid chamber transversely to the axes of the inlet port and the outlet port.

In some configurations, the angle is between about 0 degrees and about 40 degrees relative to the plane.

In some configurations, the angle is between about 10 degrees and about 40 degrees relative to the plane.

In some configurations, each grip is at a grip distance from the centre of the humidifier liquid chamber, wherein the distance is between about 50 mm and about 70 mm.

In some configurations, the grip distance is between about 55 mm and about 65 mm.

In some configurations, the grips are located on opposing sides of the humidifier liquid chamber.

In some configurations, the grips extend downwardly from the top of the humidifier liquid chamber.

In some configurations, the grips extend part way down from the top of the humidifier liquid chamber.

In some configurations, the grips comprise inward recesses.

In some configurations, the recesses are scalloped.

In some configurations, the recesses have a radius of between about 10 mm and about 20 mm.

In some configurations, the grips have substantially smooth grip surfaces.

In some configurations, the grips have textured grip surfaces.

In some configurations, the chamber has a substantially cylindrical shape.

In some configurations, the chamber tapers towards a centre of the humidifier liquid chamber from a base of the humidifier liquid chamber to a top of the humidifier liquid chamber.

In some configurations, the base is heat conductive.

In some configurations of the humidifier liquid chamber, the base of the humidifier liquid chamber is removable from a body of the humidifier liquid chamber.

In some configurations of the humidifier liquid chamber, the humidifier liquid chamber comprises one or more base removal features to assist with removing the base of the humidifier liquid chamber from the body of the humidifier liquid chamber.

In some configurations of the humidifier liquid chamber, the one or more base removal features is/are located between a lower flange of the body of the humidifier liquid chamber and an outwardly extending flange of the base of the humidifier liquid chamber.

In some configurations of the humidifier liquid chamber, the one or more base removal features comprises a slot.

In some configurations, the inlet port and outlet port are located at a front of the humidifier liquid chamber.

In some configurations, the grips are located in sides of the humidifier liquid chamber, rearward of the plane.

In some configurations, the humidifier liquid chamber is configured to engage with a liquid chamber bay of a humidifier by sliding the humidifier liquid chamber substantially linearly into the liquid chamber bay.

Additionally, in accordance with certain features, aspects and advantages of at least one of the embodiments disclosed herein, a humidifier liquid chamber is disclosed, the humidifier liquid chamber comprising:
an inlet port and an outlet port, the inlet port and the outlet port defining respective axes, wherein the inlet port and the outlet port are substantially parallel to each other and are configured to be in a substantially horizontal orientation when the humidifier liquid chamber is in use;
flow tubes extending into the humidifier liquid chamber from the inlet port and the outlet port, wherein each flow tube comprises an opening at or adjacent an end of the flow tube that is distal from the respective port, wherein the opening is configured to direct flow and is spaced from a wall of the humidifier liquid chamber, and wherein the opening in each flow tube faces a roof of the humidifier liquid chamber; and
a baffle in the interior of the humidifier liquid chamber and between the openings of the flow tubes, wherein the baffle extends downward from a roof of the humidifier liquid chamber and terminates above a maximum liquid fill level of the humidifier liquid chamber.

In some configurations, a distance between a bottom edge of the baffle and the maximum liquid fill level is between about 5 mm and about 15 mm.

Additionally, in accordance with certain features, aspects and advantages of at least one of the embodiments disclosed herein, a breathing assistance apparatus is disclosed, the breathing assistance apparatus comprising a base unit and either:
the humidifier liquid chamber as outlined above; or
the humidifier liquid chamber manifold as outlined above coupled to or integrated with a humidifier liquid chamber.

In some configurations, the humidifier liquid chamber is located in a recess of the base unit.

In some configurations, the humidifier liquid chamber is removable from the recess of the base unit.

In some configurations, the ports of the humidifier liquid chamber or the base unit connection ports of the humidifier liquid chamber manifold are engaged with complementary chamber connection ports of the base unit.

In some configurations, the complementary chamber connection ports are arranged to be received in the ports of the humidifier liquid chamber or the base unit connection ports of the humidifier liquid chamber manifold.

In some configurations, an overlapping length of the ports of the humidifier liquid chamber or the base unit connection ports of the humidifier liquid chamber manifold and the complementary chamber connection ports is sufficient to provide protection against liquid ingress.

In some configurations, the base unit comprises a plurality of seals on each of complementary chamber connection ports.

In some configurations, the base unit comprises a flow generator.

In some configurations, the base unit is configured to fluidly connect to a separate or remote flow generator.

Features from one or more embodiments or configurations may be combined with features of one or more other embodiments or configurations. Additionally, more than one embodiment may be used together during a process of respiratory support of a patient.

It is intended that reference to a range of numbers disclosed herein (for example, 1 to 10) also incorporates reference to all rational numbers within that range (for example, 1, 1.1, 2, 3, 3.9, 4, 5, 6, 6.5, 7, 8, 9 and 10) and also any range of rational numbers within that range (for example, 2 to 8, 1.5 to 5.5 and 3.1 to 4.7) and, therefore, all sub-ranges of all ranges expressly disclosed herein are hereby expressly disclosed. These are only examples of what is specifically intended and all possible combinations of numerical values between the lowest value and the highest value enumerated are to be considered to be expressly stated in this application in a similar manner.

It should be understood that alternative embodiments or configurations may comprise any or all combinations of two or more of the parts, elements or features illustrated, described or referred to in this specification.

To those skilled in the art to which the invention relates, many changes in construction and widely differing embodiments and applications of the invention will suggest themselves without departing from the scope of the invention as defined in the appended claims. The disclosures and the descriptions herein are purely illustrative and are not intended to be in any sense limiting. Where specific integers are mentioned herein which have known equivalents in the art to which this invention relates, such known equivalents are deemed to be incorporated herein as if individually set forth.

The term 'comprising' as used in this specification means 'consisting at least in part of'. When interpreting each statement in this specification that includes the term 'comprising', features other than that or those prefaced by the term may also be present. Related terms such as 'comprise' and 'comprises' are to be interpreted in the same manner.

As used herein the term '(s)' following a noun means the plural and/or singular form of that noun.

As used herein the term 'and/or' means 'and' or 'or', or where the context allows both.

The invention envisages constructions of which the following gives examples only.

### BRIEF DESCRIPTION OF THE DRAWINGS

Specific embodiments and modifications thereof will become apparent to those skilled in the art from the detailed description herein having reference to the figures that follow, of which:
Figure 1 shows in diagrammatic form a breathing assistance apparatus.
Figure 2 is a front/right side overhead perspective view of a breathing assistance apparatus with a humidifier liquid chamber positioned in the recess of the breathing assistance apparatus base unit.
Figure 3 is a front/left side overhead perspective view of the breathing assistance apparatus with the humidifier liquid chamber removed from the recess of the breathing assistance apparatus base unit.
Figure 4 is a front/right side overhead perspective view of the breathing assistance apparatus base unit with the humidifier liquid chamber and heater plate not shown.
Figure 5 is a front/right side bottom perspective view of the breathing assistance apparatus base unit with the humidifier liquid chamber not shown.
Figure 6 is a front/right side overhead perspective view of a removable elbow and shroud of the breathing assistance apparatus base unit.
Figure 7 is a right side view of the elbow.
Figure 8 is a rear/right side perspective view showing the elbow from below, and with the seal not shown.
Figure 9 is a top plan view of the shroud and elbow, prior to insertion of the elbow into the shroud.
Figure 10 is an enlarged right side view showing engagement features on the elbow engaging with the shroud (broken lines).
Figure 11 is a right side view of the elbow showing seals and the location of temperature sensor(s) in broken lines.
Figure 12 is a right side view of part of the elbow showing details of the seals.
Figure 13 is an overhead perspective view of the humidifier liquid chamber.
Figure 14 is an overhead plan view of the humidifier liquid chamber.
Figure 15 is a front view of the humidifier liquid chamber.
Figure 16 is a left side sectional view of the humidifier liquid chamber along line 16-16 of Figure 15.
Figure 17 is an overhead perspective view of one of the flow tubes of the humidifier liquid chamber.
Figure 18 is a front view of the humidifier liquid chamber showing an internal baffle in broken lines.
Figure 19 is a left side sectional view of the humidifier liquid chamber along the line 19-19 of Figure 18.
Figure 20 is a front/left side overhead perspective view of the breathing assistance apparatus base unit and humidifier liquid chamber, prior to insertion of the humidifier liquid chamber into the recess of the breathing assistance apparatus base unit.
Figure 21 is a front/left side overhead perspective view similar to Figure 20, but with the humidifier liquid chamber inserted into the recess of the breathing assistance apparatus base unit.
Figure 22 is a right side sectional view showing the interaction of the seals on one of the apparatus ports with one of the humidifier liquid chamber ports.
Figure 23 is a flow diagram from a computational fluid dynamics model showing the effect of humidifier liquid chamber baffle height on flow stagnation around the humidifier liquid chamber inlet for a humidifier liquid chamber with no gap between the baffle and the liquid in the chamber (left side), and for a humidifier liquid chamber with a 10 mm gap between the baffle and the liquid in the chamber (right side).
Figure 24 is a left side view of the humidifier liquid chamber showing a moment M created by an offset grip relative to the liquid chamber central axis.
Figure 25 is an overhead plan view of the humidifier liquid chamber showing dimensions for a thumb abducted large diameter grasp.
Figure 26 is an overhead perspective view of a humidifier liquid chamber and manifold assembly for use with the breathing assistance apparatus.
Figure 27 is an overhead plan view of the manifold.
Figure 28 is an overhead perspective view of the humidifier liquid chamber.
Figure 29 is a side part sectional view through one of the chamber connection ports of the manifold, showing its connection to one of the ports of the humidifier liquid chamber.
Figure 30 is an overhead perspective view of an alternative configuration humidifier liquid chamber with a removable base.
Figure 31 is an inverted side view of the alternative configuration humidifier liquid chamber with the base removed.
Figure 32 is a left side sectional view similar to Figure 19, showing an alternative baffle configuration of the humidifier liquid chamber.

### DETAILED DESCRIPTION OF SPECIFIC EMBODIMENTS

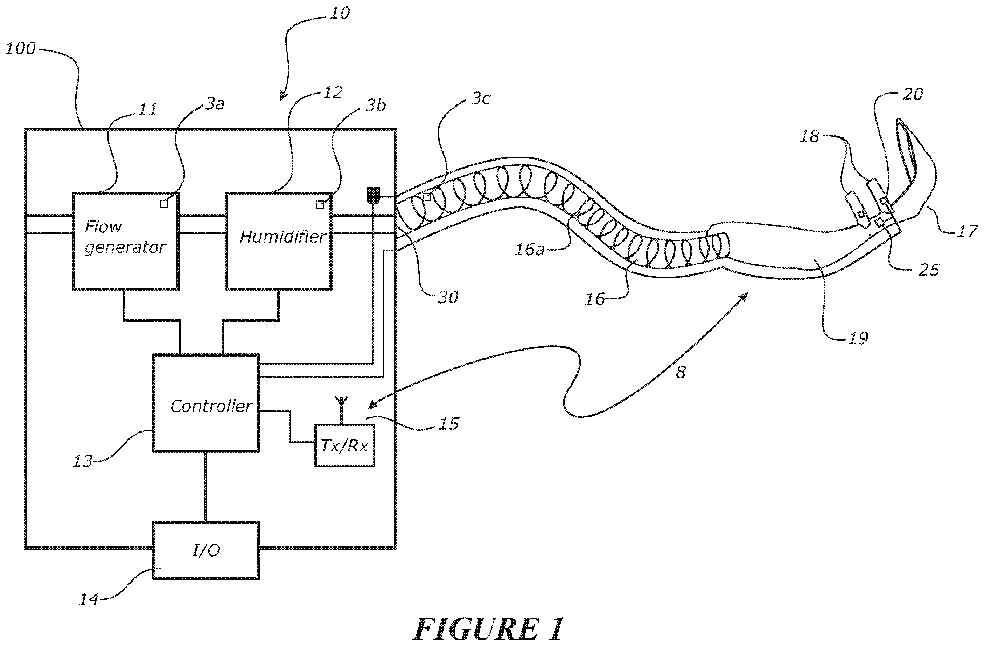

A breathing assistance apparatus 10 for delivering a flow of gas (which may contain one or more gases) to a patient is shown in Figure 1. The apparatus 10 could, for example, be a CPAP apparatus or a high flow apparatus. An exemplary CPAP apparatus is described in WO 2011/056080.

A CPAP apparatus is a gases supply and optionally gases humidification apparatus. The apparatus is operable to provide respiratory assistance to patients or users who require a supply of gas (humidified or otherwise) at positive pressure for the treatment of diseases such as Obstructive Sleep Apnea (OSA), snoring, or Chronic Obstructive Pulmonary Disease (COPD) and the like. A CPAP apparatus would typically include a humidifier liquid chamber, so as to form a combined assisted breathing unit and humidifier.

CPAP apparatuses, when used with a humidifier, typically have a structure where gases at a required pressure are delivered from an assisted breathing unit or blower unit to a humidifier liquid chamber downstream from the blower. As the gases pass through the humidifier liquid chamber, they become saturated with liquid vapour (e.g. water vapour). A flexible tubular gases conduit delivers the gases to a user or patient downstream from the humidifier liquid chamber.

A high flow apparatus may be used to deliver a high gas flow or high flow therapy to a patient to assist with breathing and/or treat breathing disorders including chronic obstructive pulmonary disease (COPD). A high flow apparatus includes a gases supply and typically includes a humidification apparatus.

The breathing assistance apparatuses typically have one or more accessories such as a breathing conduit and a patient interface such as a cannula or mask for delivering gases to a patient. The conduit enables gases to be delivered from the housing of the breathing assistance apparatus to the patient. For example, the apparatus may be placed on a floor or other support surface, and the patient may be in a bed. The breathing assistance apparatus may have a recess for receipt of a humidifier liquid chamber. The humidifier liquid chamber will receive liquid from, for example, a flexible liquid bag that delivers liquid to a humidifier liquid chamber via one or more tubes. Alternatively, the humidifier liquid chamber can be removed and refilled as required. The recess will contain a heater plate to heat the humidifier liquid chamber, to humidify gases passing through the humidifier liquid chamber. The humidified gases are then delivered to the patient.

In general terms, the apparatus 10 comprises a main housing 100 that contains a flow generator 11 in the form of a motor/impeller arrangement, a humidifier 12, a controller 13, and a user I/O interface 14 (comprising, for example, a display and input device(s) such as button(s), a touch screen, or the like). The controller 13 is configured or programmed to control the components of the apparatus, including: operating the flow generator 11 to create a flow of gas (gas flow) for delivery to a patient, operating the humidifier 12 to humidify and/or heat the generated gas flow, receive user input from the user interface 14 for reconfiguration and/or user-defined operation of the apparatus 10, and output information (for example on the display) to the user. The user could be a patient, healthcare professional, or anyone else interested in using the apparatus.

A patient breathing conduit 16 is connected to a gas flow output or patient outlet port 30 in the housing 100 of the breathing assistance apparatus 10, and is connected to a patient interface 17 such as a nasal cannula with a manifold 19 and nasal prongs 18. Additionally, or alternatively, the patient breathing conduit 16 could be connected to a face mask. Additionally, or alternatively, the patient breathing conduit could be connected to a nasal pillows mask, and/or a nasal mask, and/or a tracheostomy interface, or any other suitable type of patient interface. The gas flow, which may be humidified, that is generated by the breathing assistance apparatus 10 is delivered to the patient via the patient breathing conduit 16 through the patient interface 17. The patient breathing conduit 16 can have a heater wire 16a to heat gas flow passing through to the patient. The heater wire 16a is under the control of the controller 13. The patient breathing conduit 16 and/or patient interface 17 can be considered part of the breathing assistance apparatus 10, or alternatively peripheral to it. The breathing assistance apparatus 10, breathing conduit 16, and patient interface 17 may together form a breathing assistance system or, in some configurations, a flow therapy system.

General operation of an exemplary breathing assistance apparatus 10 will be known to those skilled in the art, and need not be described in detail here. However, in general terms, the controller 13 controls the flow generator 11 to generate a gas flow of the desired flow rate, controls one or more valves to control the mix of air and oxygen or other alternative gas, and/or controls the humidifier 12 to humidify the gas flow and/or heat the gas flow to an appropriate level. The gas flow is directed out through the patient breathing conduit 16 and patient interface 17 to the patient. The controller 13 can also control a heating element in the humidifier 12 and/or the heating element 16a in the patient breathing conduit 16 to humidify and/or heat the gas to a desired temperature that achieves a desired level of therapy and/or comfort for the patient. The controller 13 can be programmed with, or can determine, a suitable target temperature of the gas flow.

Operation sensors 3a, 3b, 3c, 20, and 25, such as flow, temperature, humidity, and/or pressure sensors, can be placed in various locations in the breathing assistance apparatus 10 and/or the patient breathing conduit 16 and/or patient interface 17. Output from the sensors can be received by the controller 13, to assist it to operate the breathing assistance apparatus 10 in a manner that provides optimal therapy. In some configurations, providing optimal therapy includes meeting a patient's inspiratory flow. The apparatus 10 may have a transmitter and/or receiver 15 to enable the controller 13 to receive signals 8 from the sensors and/or to control the various components of the breathing assistance apparatus 10, including but not limited to the flow generator 11, humidifier 12, and heater wire 16a, or accessories or peripherals associated with the breathing assistance apparatus 10. Additionally, or alternatively, the transmitter and/or receiver 15 may deliver data to a remote server or enable remote control of the apparatus 10.

The breathing assistance apparatus 10 may be any suitable type of apparatus, but in some configurations may deliver a high gas flow or high flow therapy (of e.g. air, oxygen, other gas mixture, or some combination thereof) to a patient to assist with breathing and/or treat breathing disorders. In some configurations, the gas is or comprises oxygen. In some configurations, the gas comprises a blend of oxygen and ambient air. High flow therapy as discussed herein is intended to be given its typical ordinary meaning as understood by a person of skill in the art which generally refers to a respiratory assistance system delivering a targeted flow of humidified respiratory gases via an intentionally unsealed patient interface with flow rates generally intended to meet or exceed inspiratory flow of a patient. Typical patient interfaces include, but are not limited to, a nasal or tracheal patient interface. Typical flow rates for adults often range from, but are not limited to, about fifteen liters per minute (LPM) to about seventy liters per minute or greater. Typical flow rates for pediatric patients (such as neonates, infants and children) often range from, but are not limited to, about one liter per minute per kilogram of patient weight to about three liters per minute per kilogram of patient weight or greater. High flow therapy can also optionally include gas mixture compositions including supplemental oxygen and/or administration of therapeutic medicaments. High flow therapy is often referred to as nasal high flow (NHF), humidified high flow nasal cannula (HHFNC), high flow nasal oxygen (HFNO), high flow therapy (HFT), or tracheal high flow (THF), among other common names.

For example, in some configurations, for an adult patient 'high flow therapy' may refer to the delivery of gases to a patient at a flow rate of greater than or equal to about 10 liters per minute (10 LPM), such as between about 10 LPM and about 100 LPM, or between about 15 LPM and about 95 LPM, or between about 20 LPM and about 90 LPM, or between about 25 LPM and about 85 LPM, or between about 30 LPM and about 80 LPM, or between about 35 LPM and about 75 LPM, or between about 40 LPM and about 70 LPM, or between about 45 LPM and about 65 LPM, or between about 50 LPM and about 60 LPM. In some configurations, for a neonatal, infant, or child patient 'high flow therapy' may refer to the delivery of gases to a patient at a flow rate of greater than 1 LPM, such as between about 1 LPM and about 25 LPM, or between about 2 LPM and about 25 LPM, or between about 2 LPM and about 5 LPM, or between about 5 LPM and about 25 LPM, or between about 5 LPM and about 10 LPM, or between about 10 LPM and about 25 LPM, or between about 10 LPM and about 20 LPM, or between about 10 LPM and 15 LPM, or between about 20 LPM and 25 LPM. A high flow therapy apparatus with an adult patient, a neonatal, infant, or child patient, may, in some configurations, deliver gases to the patient at a flow rate of between about 1 LPM and about 100 LPM, or at a flow rate in any of the sub-ranges outlined above. Gases delivered may comprise a percentage of oxygen. In some configurations, the percentage of oxygen in the gases delivered may be between about 20% and about 100%, or between about 30% and about 100%, or between about 40% and about 100%, or between about 50% and about 100%, or between about 60% and about 100%, or between about 70% and about 100%, or between about 80% and about 100%, or between about 90% and about 100%, or about 100%, or 100%.

High flow therapy has been found effective in meeting or exceeding the patient's inspiratory flow, increasing oxygenation of the patient and/or reducing the work of breathing. Additionally, high flow therapy may generate a flushing effect in the nasopharynx such that the anatomical dead space of the upper airways is flushed by the high incoming gas flows. This creates a reservoir of fresh gas available for each and every breath, while minimising re-breathing of carbon dioxide, nitrogen, etc.

In one example for high flow therapy, an unsealed or non-sealing user interface, e.g. a nasal cannula, is used. For CPAP a sealed interface is typically used, e.g. a nasal mask, full face mask, or nasal pillows.

The patient interface may be a non-sealing interface to prevent barotrauma (e.g. tissue damage to the lungs or other organs of the respiratory system due to difference in pressure relative to the atmosphere). The patient interface may be a nasal cannula with a manifold and nasal prongs, and/or a face mask, and/or a nasal pillows mask, and/or a nasal mask, and/or a tracheostomy interface, or any other suitable type of patient interface.

As described below, the breathing assistance apparatus 10 has various features to assist with the functioning, use, and/or configuration of the apparatus 10.

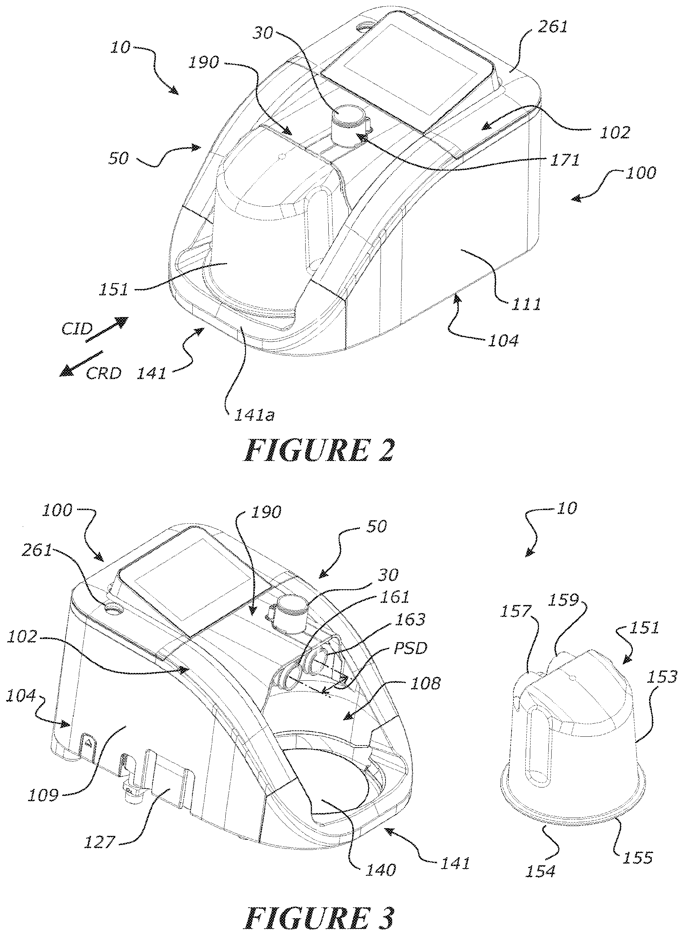

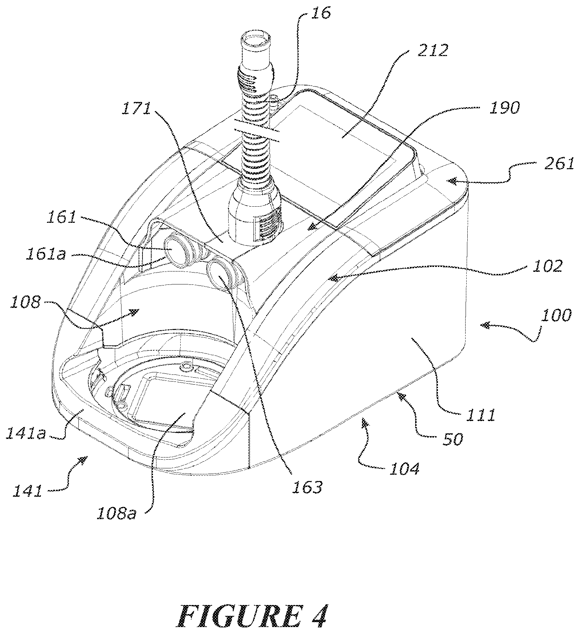

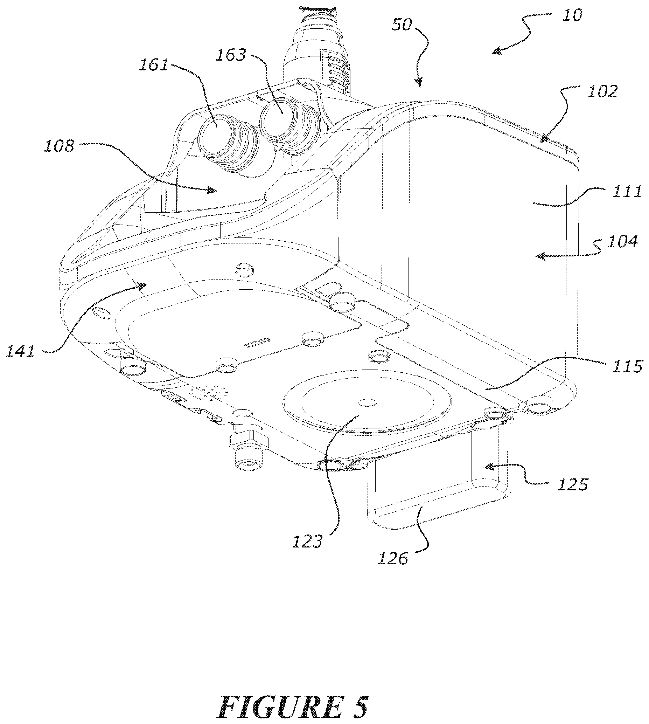

As shown in figures 2 to 5, a first configuration breathing assistance apparatus 10 comprises a breathing assistance apparatus base unit 50 having a main housing 100. The main housing 100 has a main housing upper chassis 102 and a main housing lower chassis 104.

The main housing of the base unit 50 has a peripheral wall arrangement. The peripheral wall arrangement defines a recess 108 that provides a humidifier liquid chamber bay for receipt of a removable humidifier liquid chamber 151. The removable humidifier liquid chamber 151 contains a suitable liquid such as water for humidifying gases that will be delivered to a patient.

The base unit 50 of the apparatus 10 may have a movable finger guard 141 that guards against a user touching a base flange 155 of the humidifier liquid chamber when the humidifier liquid chamber is in place in the recess 108 and when a barrier 141a of the finger guard is in a covering position as shown in the figures. The barrier 141a is movable between the covering position and a lowered access position in which the recess 108 is less covered or is uncovered by the barrier 141a.

In the form shown, the main housing lower chassis 104 peripheral wall arrangement comprises a substantially vertical left side outer wall 109 that is oriented in a front-to-rear direction of the main housing 100, a substantially vertical right side outer wall 111, and a substantially vertical rear outer wall. A bottom wall 115 extends between and connects the lower ends of the left side outer wall 109, the right side outer wall 111, and the rear wall, and forms a base of the apparatus and a substantially horizontal floor portion of the liquid chamber bay.

The floor portion of the recess 108 has a receptacle portion 108a to receive a heater arrangement such as a heater plate 140 or other suitable heating element(s) for heating liquid in the humidifier liquid chamber 151 for use during a humidification process. The heater plate would typically have a shape that substantially corresponds to the shape of a base 154 of the humidifier liquid chamber 151, such as a circular shape for example. The heater plate 140 is resiliently mounted; for example, on biasing device(s) such as spring(s). The resilient mounting enables the heater plate to move downwardly to accommodate the humidifier liquid chamber 151 in the recess 108, while maintaining good contact between the heater plate 140 and the base of the humidifier liquid chamber once the humidifier liquid chamber is inserted in the recess 108.

The main housing lower chassis 104 is attachable to the upper chassis 102, either by suitable fasteners or integrated attachment features such as clips for example. When the main housing lower chassis 104 is attached to the main housing upper chassis 102, the walls of the upper and lower chassis engage with each other.

The lower chassis 104 has a motor recess for receipt of a motor module which may be permanently inserted in the recess or may be removable from the recess. A recess opening is provided in the bottom wall 115 adjacent a rear edge thereof, for receipt of the removable motor module. A base 123 of the motor module covers the opening into the motor recess 121. The motor module comprises a motor that forms a blower to cause gas flow, and may comprise one or more sensors to sense properties of the gas passing through the motor module. The motor module may comprise sensor(s) to sense parameters of gases flowing through the motor module.

The motor module and housing of the base unit of the apparatus 10 are provided with suitable tubes and/or gas flow passages to deliver gases from one or more gases inlets of the base unit 50, to a gas inlet port 157 of the humidifier liquid chamber 151 to humidify the gases. The gases are delivered from a gas outlet port 159 of the humidifier liquid chamber 151 to the patient outlet port 30 (via a humidified gas inlet port 163) and thereby to the patient via the patient breathing conduit 16 and patient interface 17.

The motor recess 122 comprises a recess opening in a bottom wall 115 of the housing. Alternatively, the recess opening could be in a different part of the housing, such as a side, front, or top of the housing.

The base unit 50 of the apparatus 10 may have a battery module 125 to provide power to the apparatus when there is a power outage or for portable use. The battery module comprises a battery cover 126 containing a battery. The battery of the battery module 125 may be replaceable.

In the form shown, the battery cover 126 of the battery module 125 is coupled to an exterior of the back wall of the apparatus. This provides a large surface area to cool the battery and reduces the amount of heat entering the housing from the battery. Additionally, this configuration reduces the influence of heat generated by components of the apparatus on the battery, particularly when the battery is being charged. In an alternative configuration, the battery may be internally mounted in the main housing.

The housing may be provided with a battery cover 126 to cover the battery once installed. Alternatively, the battery may mount directly to the housing 100 without a cover. The battery, and therefore the battery cover 126, may be sized to not extend beyond the bottom wall 115 of the housing. Alternatively, the battery cover 126 may be longer and extend beyond the bottom wall 115 of the housing to accommodate a larger battery.

As shown in figure 3, the base unit 50 of the apparatus 10 has a mounting feature 127 for mounting the apparatus to a support apparatus.

The mounting feature 127 may be integrally formed with part of the main housing of the base unit 50 of the apparatus 10. In the form shown, the mounting feature 127 is integrally formed with the left side wall 109 the lower chassis 104 of the housing. The mounting feature 127 could instead be integrally formed with any of the other walls of the housing, such as a rear wall, right side wall, or other wall.

The main housing of the apparatus may be formed from any suitable material that will allow the mounting feature 127 to be integrally formed. For example, the housing may be formed from polycarbonate.

The integral mounting feature 127 has greater impact strength compared to an additional, screwed in part. Strengthening of the mounting feature 127 may also be done by, for example, varying the wall thickness, ribbing, or varying in internal geometries.

The housing comprises a handle 261 connected to the upper chassis 102 of the housing 100. The handle 261 is movable between a lowered storage position and a raised carrying position. In the raised carrying position, a user can use the handle to carry the apparatus 10.

Figures 3, 13-22, and 23-25 show a humidifier liquid chamber 151 for use with the breathing assistance apparatus 10. The humidifier liquid chamber 151 is a removable liquid chamber to be filled with liquid such as water for the humidification of respiratory gases. The humidifier liquid chamber 151 is removable from the base unit 50 of the breathing assistance apparatus 10 to be more easily re-filled or disposed of.

The humidifier liquid chamber 151 has a body 152 having a peripheral wall 153 and a roof 156. The roof 156 is at the top of the liquid chamber. The body 152 defines an internal chamber for receipt of a liquid. A base 154 is provided at the lower end of the peripheral wall 153, and comprises a base flange 155 that projects outwardly from the lower end of the peripheral wall 153. First and second base unit connection ports comprising a liquid chamber gas inlet port 157 and a liquid chamber gas outlet port 159 are in communication with the internal chamber of the humidifier liquid chamber 151. The breathing assistance apparatus base unit 50 comprises complementary chamber connection ports comprising a gas outlet port 161 and a humidified gas inlet port 163. When the humidifier liquid chamber 151 is received in the recess 108 to engage with the housing 100 of the base unit 50, the liquid chamber gas inlet port 157 connects to the gas outlet port 161 that receives gases from the motor module via a gasflow passage, and the liquid chamber gas outlet port 159 connects to the humidified gas inlet port 163 to deliver humidified gases from the humidifier liquid chamber to the patient outlet port 30.

The humidifier liquid chamber 151 could have a generally circular peripheral shape, or could be any other suitable shape, with the recess 108 shape modified accordingly if required.

In the form shown, the humidifier liquid chamber 151 has a substantially cylindrical shape.

The humidifier liquid chamber tapers towards a centre C of the humidifier liquid chamber from a base of the humidifier liquid chamber to a top of the humidifier liquid chamber. The body 152 of the humidifier liquid chamber 151 resembles a truncated cone and is generally formed from clear plastic material to aid the user to observe the fill level.

The base 154 of the humidifier liquid chamber is heat conductive. In particular, the base 154 of the humidifier liquid chamber 151 is made from a highly heat conductive material, which allows heating of the liquid in the humidifier liquid chamber when in contact with the heater plate 140 of the base unit 50 of the breathing assistance apparatus 10 during use.

The humidifier liquid chamber 151 can be fluidly coupled to the base unit 50 of the apparatus 10 in a rearward insertion direction CID of the humidifier liquid chamber 151 into the recess 108, from a position at the front of the housing 100 in a direction toward the rear of the housing 100. The gas outlet port 161 is in fluid communication, via a fixed L shaped elbow, with a gas flow passage from the motor/impeller unit.

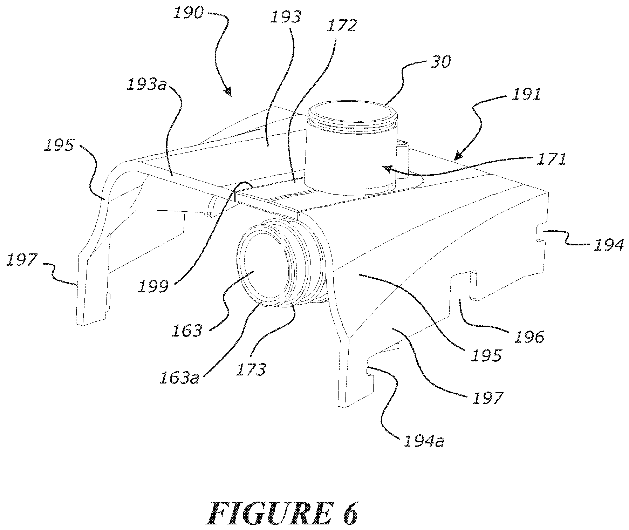

The humidified gas inlet port 163 is embodied in a removable component comprising removable elbow 171 (figures 6 to 12) that can be removably connected to the housing. The removable elbow 171 is L-shaped, and further comprises the upstanding patient outlet port 30 for coupling to the patient breathing conduit 16 to deliver gases to the patient interface 17. In different configurations, the removable component may not have an elbow shape, and could instead, for example, have aligned inlet and outlet ports.

The gas outlet port 161, humidified gas inlet port 163, and patient outlet port 30 each comprise soft seals such as wiper seals, L-seals, X-rings, or O-rings to provide a sealed gases passageway between the apparatus 10, the humidifier liquid chamber 151, and the patient breathing conduit 16 and optionally one or more other accessories.

The gas outlet port 161 and gas inlet port 163 comprise multiple sealing elements. The sealing elements may be wiper seals, L-seals, X-rings, or O-rings. The wiper seals may have a T-shaped cross-section. The gas outlet port 161 and the gas inlet port 163 may each comprise two, three, or more sealing elements. In one configuration, each of the gas inlet port 163 and gas outlet port 161 comprises a pair of wiper seals. In this configuration, the gas inlet port 163 has two wiper seals positioned adjacent each other on the gas inlet port 163. Similarly, the gas outlet port 161 comprises a pair of wiper seals positioned adjacent each other on the gas outlet port 161. The pair of wiper seals (or of the other types of sealing elements) on each port 161, 163 improves the seal with the corresponding base unit connection ports 157, 159 and provides improved protection against liquid ingress into the interior of the housing of the base unit 50 of the apparatus where electronics are located.

When the humidifier liquid chamber 151 is coupled to the gas inlet port 163 and gas outlet port 161 of the base unit 50, one wiper seal may be positioned inside each base unit connection port 157, 159 and one wiper seal may be located outside each base unit connection port 157, 159, when the humidifier liquid chamber is assembled with the base unit 50. Alternatively, both wiper seals are positioned inside the respective base unit connection ports 157, 159 when the humidifier liquid chamber 151 is assembled onto the heater plate 140 in the recess 108. The arrangement of using two wiper seals per port 161, 163 provides redundancy for liquid ingress. Similar arrangement can be used for L-seals, X-rings, or O-rings. The gas outlet port 161 and gas inlet port 163 of the base unit 50 are structured to have an elongate portion; i.e., a length of the ports 161, 163 is such that the wiper seals, L- seals, X-rings, or O-rings are retained on the ports 161, 163.

The gas inlet port 157 of the humidifier liquid chamber is complementary with the gas outlet port 161 of the breathing assistance apparatus base unit 50, and the gas outlet port 159 of the humidifier liquid chamber is complementary with the humidified gas inlet port 163 of the breathing assistance apparatus base unit 50. The axes of those ports may be parallel and/or horizontal enable the humidifier liquid chamber 151 to be inserted into the recess 108 of the base unit 50 in a substantially linear movement to form gas connections between the ports.

The chamber connection ports 161, 163 are parallel cylindrical features extending from the housing of the breathing assistance apparatus base unit 50. The ports 161, 163 will typically have an equal profile, and equal length, and axes located on the same horizontal plane. The ports 161, 163 will typically terminate on the same vertical plane at their distal ends. The ports 161, 163 have a port separation distance PSD or pitch (Figure 3), which is the horizontal distance between the centre or axis of each port 161, 163. This is substantially equal to the horizontal distance between the centres of the base unit connection ports 157, 159 of the humidifier liquid chamber.

The chamber connection ports 161, 163 (which in the form shown are male connection members) of the breathing assistance apparatus base unit 50 insert into the base unit connection ports 157, 159 (which in the form shown are female connection members) of the liquid chamber in a concentric manner. Therefore, each base unit connection port 157, 159 of the humidifier liquid chamber is configured to receive a corresponding chamber connection port 161, 163 of the breathing assistance apparatus base unit 50. The inner diameter of the base unit connection ports 157, 159 is larger than the outer diameter of the chamber connection ports 161, 163.

The chamber connection ports 161, 163 and base unit connection ports 157, 159 can be cylindrical. The ports 157, 159, 161, 163 can have any suitable cross-sectional shape.

The humidifier liquid chamber 151 may initially be inserted into the recess 108 on an angle, and then tilted to be substantially horizontal, so that a rear part of movement of the humidifier liquid chamber 151 is substantially linear. The recess 108 may comprise one or more retaining features such as guide rails for example, to assist with holding the humidifier liquid chamber in position in the recess 108.

The breathing assistance apparatus 10 may have any one or more of the features and/or functionality of the breathing assistance apparatus described and shown in WO2016/207838A9.

In order to prevent or minimise gas leaking from either of the two connections (port 157 to port 161, and port 159 to port 163), one or more sealing elements are provided for each connection. The one or more sealing elements may be on the outer surface of male ports, and seal against the inner surface of female ports. In one configuration, the gas inlet port 157 and the gas outlet port 159 of the humidifier liquid chamber are the female ports, and the housing ports, i.e. the gas outlet port 161 and the humidified gas inlet port 163, are the male ports. Alternatively, the ports 157, 159 of the humidifier liquid chamber may be the male ports and the ports 161, 163 of the breathing assistance apparatus base unit 50 may be the female ports.

Figures 6 to 12 show details of the removable elbow 171. Although this section describes the features of the humidified gas inlet port 163 and its interaction with the gas outlet port 159 of the humidifier liquid chamber, including a seal 173, the features of the gas outlet port 161 of the housing and its interaction with the gas inlet port 157 of the humidifier liquid chamber will be the same.

The humidified gas inlet port 163 comprises a generally horizontally oriented extended portion 162 that is configured to insert within the gas outlet port 159 of the humidifier liquid chamber. The terminal end 163a of the port has a rounded edge to aid in aligning the gas outlet port 159 with the humidified gas inlet port 163. Additionally, the terminal end 163a is slightly smaller in diameter than the gas outlet port 159.

At least one recessed portion 163b is provided on the port 163. This recessed portion allows a seal 173 to be attached to the port. The seal 173 can be attached by being overmoulded directly onto the port 163. Alternatively, the seal 173 can be stretched over the terminal end 163a of the port in order to place it in the recessed portion 163b. The seal may be shaped so that it remains in a stretched state once it has been placed in the recessed portion, to assist with maintaining the seal in position. Once the seal 173 is located in the recessed portion 163b, the boundaries of the recessed portion 163b prevent any movement of the seal 173 along the port 163. This allows the humidifier liquid chamber 151 to be connected/disconnected in a lateral motion without dislodging the seal 173 from the port 163.

The humidified gas inlet port 163 may comprise a plurality of seals or sealing elements located in the recessed portion 163b. The plurality of seals 175 may be a pair of wiper seals, L-seals, X-rings, or O-rings. The wiper seals may have a T-shaped cross-section. In some configurations, the gas inlet port 163 may comprise three or more seals or sealing elements. A similar seal arrangement can also be on the outlet port 161 of the base unit 50. The wiper seals, i.e. double seals, prevent or reduce breathing gas leak and/or condensate from moving towards the electronics in the removable elbow 171 and the electrical connector 178 (described below) of the elbow. Similarly, the seals reduce the chance of, and preferably prevent, liquid, i.e. condensate, from moving and dripping back into the gas outlet port 161 of the base unit 50 to prevent water ingress into the electronics chamber of the base unit.

The seal 173 may be made from silicone rubber. In an alternative configuration, the seal 173 could be made from any suitable elastomer, such as polyurethane. Alternatively, the seal 173 may be made from thermoplastic elastomer(s) and/or thermoplastic vulcanisate(s), particularly if the seal will be overmoulded onto the removable elbow.

As discussed above, a plurality of sealing elements may be provided on the port 163 in order to seal against the port 159 of the humidifier liquid chamber at a plurality of locations. The plurality of sealing elements could be achieved by having a plurality of seals 173, with the port 163 having a corresponding plurality of recessed portions 163b to accommodate each seal. Alternatively, in the configuration shown, the plurality of sealing elements 175 are incorporated into a single seal 173 that is located in a single recessed portion 163b.

Having a plurality of sealing elements 175 on a single seal 173 is preferable to having a plurality of seals on the port 163, as it reduces the number of seals that need to be attached during manufacturing. Additionally, the increased width of the seal when providing a plurality of sealing elements 175 reduces the chance of the seal turning inside out during assembly onto the port 163.

Having a plurality of sealing elements 175 is beneficial in providing redundancy in the seal between the breathing assistance apparatus base unit 50 and the humidifier liquid chamber 151. This reduces the chance of a breathing gas leak and/or liquid moving into the base unit 50 occurring as it would require the seals provided by each of the sealing elements 175 to fail. Additionally, having one sealing element 175, i.e. a forward sealing element, closer to the terminal end 163a of the port 163 (compared to a single centrally-located sealing element) allows a seal to be formed between the humidifier liquid chamber 151 and the breathing assistance apparatus 10 even if the humidifier liquid chamber is not fully connected onto the port 163. Having a plurality of sealing elements 175 also helps to align the humidifier liquid chamber 151 in the recess 108 and align the ports 159, 163, by providing multiple points of contact between the ports 159, 163. The rearmost sealing element 175 on the port 163 limits the position of the humidifier liquid chamber 151 more than the forward sealing element on the port. That is because the rearmost sealing element is further from the centre of the humidifier liquid chamber 151, so tolerance between that sealing element and the liquid chamber port 159, 163 equates to a smaller available amount of angular rotation of the humidifier liquid chamber 151.

When a plurality of sealing elements is provided, the sealing elements may be spaced equally apart with respect to each other along the base 173a. In other configurations, the distance between the sealing elements may not be equal.

In some configurations, the distance between the sealing elements is equal to the distance between the forward sealing element and the terminal end 163a of the gas inlet port 163. In other configurations, the distance between the sealing elements may not be equal to the distance between the forward sealing element 175 and the terminal end 163a.

The forward sealing element is the primary sealing element, and the rearmost sealing element is the secondary sealing element. The sealing elements create an effective outer diameter of the ports 161, 163 of the base unit 50 which is slightly larger than the inner diameter of the ports 157, 159 of the humidifier liquid chamber, prior to connection.

The elastomeric nature of the seals allows for connection between the rigid bodies of the ports 157, 159, 161, 163. Having multiple sealing elements 175 allows for a pneumatic seal between the base unit 50 of the breathing assistance apparatus 10 and the humidifier liquid chamber 151, even if one seal fails.

The degree to which a successful seal is created is dependent on the depth of the base unit connection port 157, 159 of the humidifier liquid chamber into which the corresponding chamber connection port 161, 163 of the breathing assistance apparatus base unit 50 locates so that one or more seal elements 175 are engaged. This assumes the diameter of the chamber connection ports 161, 163, base unit connection ports 157, 159, and seals 175 are of appropriate dimensions to allow one or more seals to engage. Advantageously, both seals 175 are engaged to minimise any possible rotation or rocking of the humidifier liquid chamber 151, by constraining an additional degree of freedom, during use as well as to provide the pneumatic seal. Engaging both seals 175 also provides redundancy if a singular seal were to fail.

It should be understood that while a complete seal between the base unit connection ports 157, 159 of the humidifier liquid chamber and the chamber connection ports 161, 163 of the breathing assistance apparatus base unit 50, some leakage may be accommodated between those components while still providing sufficient gas flow to a user.

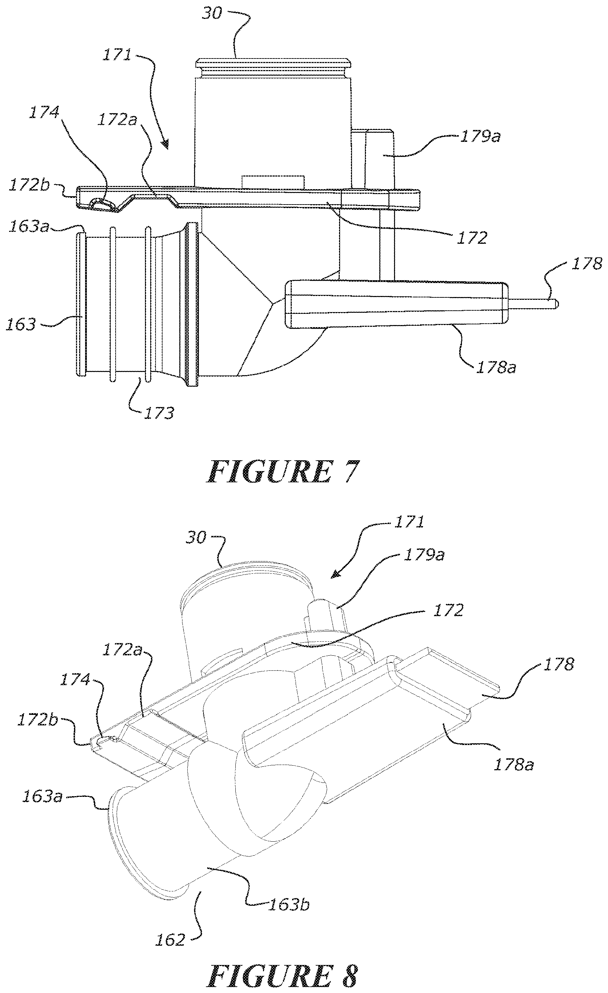

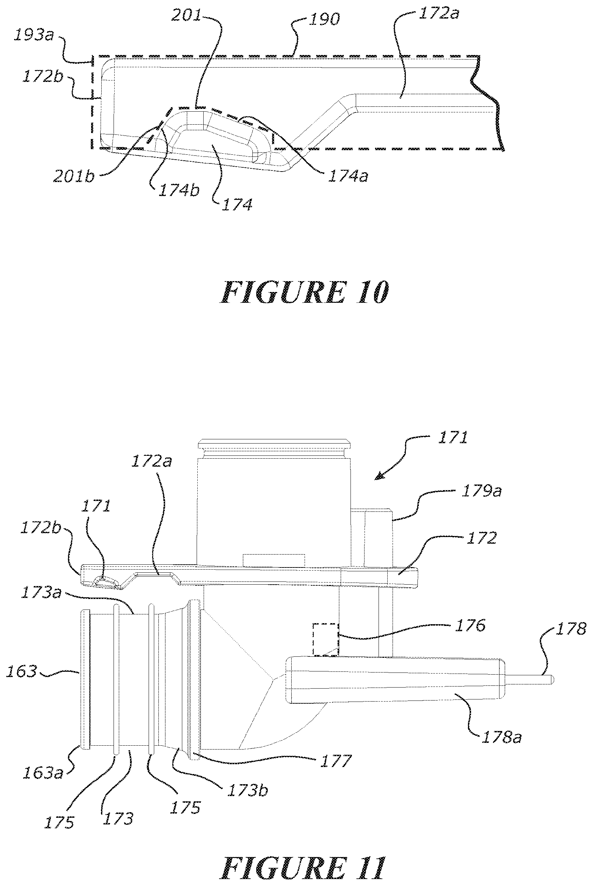

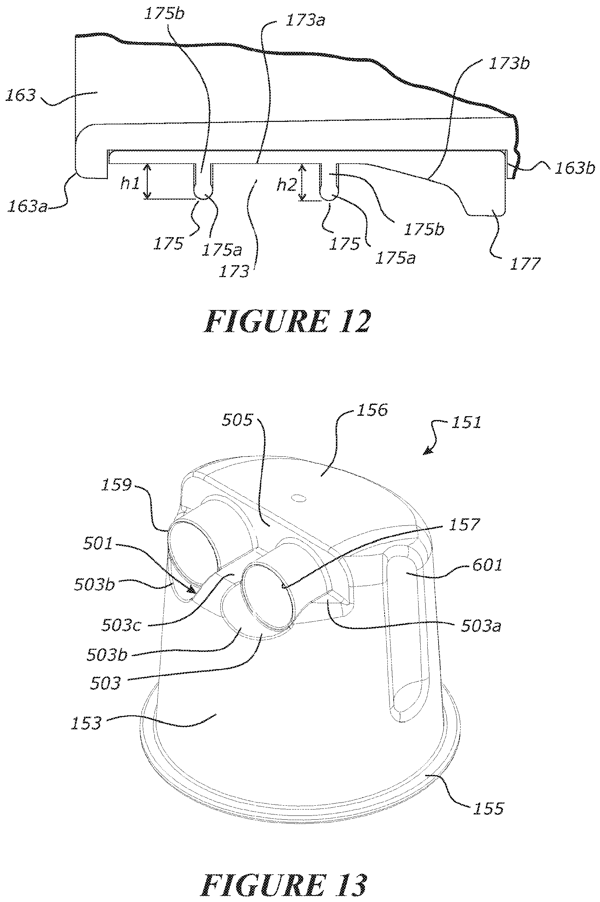

One or more of the sealing elements 175 may be a wiper seal. In the form shown in figures 7 and 11, the wiper seal is a flexible annular rim that runs around the circumference of the port 163. As shown in the cross-section in figure 12, the wiper seal has a bulbous radially outwardly located tip 175a which may have a circular cross-section for example. The tip 175a is configured to contact the inner surface of the base unit connection port 159 of the humidifier liquid chamber. The wiper seal may advantageously have a narrowed web section 175b leading from the base of the seal to the tip 175a. The narrowed web section 175b allows the wiper seal to flex more easily, while the enlarged bulbous tip 175a provides a larger surface area for the sealing contact with the inner surface of the base unit connection port 159 of the humidifier liquid chamber. In some configurations, the wiper seal may not have the bulbous tip 175a.

Referring to figure 12, the radial height h1, h2 of each wiper seal is such that in an unflexed position, the diameter of the wiper seal is greater than the inner diameter of the base unit connection port 159 of the humidifier liquid chamber. When the humidifier liquid chamber 151 is inserted into the recess 108, the wiper seal will contact the inner wall of the base unit connection port 159 of the humidifier liquid chamber and will flex downwards towards the base 173a of the seal 173 to accommodate the smaller inner diameter of the base unit connection port 159 of the humidifier liquid chamber. The elasticity of the seal will mean that the wiper seal 175 resists this flexing, thereby providing a sealing force between the points of contact on the base unit connection port 159 of the humidifier liquid chamber and the tip 175a of the wiper seal. This configuration accommodates slight variations in sizes of both the wiper seals 175 and the port 159 of the humidifier liquid chamber due to manufacturing tolerances, as the wiper seals would flex to accommodate the exact diameter of the port of the humidifier liquid chamber.

Another advantage of the wiper seals 175 is the low resistance they provide to axial movement. Unlike other seals, the wiper seals' ability to flex and conform to the inner surface of the base unit connection port of the humidifier liquid chamber results in a low amount of friction, thereby making it easier to connect and disconnect the humidifier liquid chamber 151 from the housing 100 of the base unit 50 of the breathing assistance apparatus 10.

A wiper seal 175 does, however, provide slightly more resistance to humidifier liquid chamber 151 movement in one scenario. When the humidifier liquid chamber is being moved in a first direction (e.g. towards the housing 100 in an insertion direction CID when being connected), the wiper seal 175 will be flexed in a first orientation. When the humidifier liquid chamber is being moved in a second direction (e.g. away from the housing 100 in a removal direction CRD when being disconnected), the wiper seal 175 will be flexed in a second orientation. Therefore, when the humidifier liquid chamber 151 first moves in one direction after moving in the other direction, the wiper seal 175 will also swap from one orientation to the other. This occurs by the wiper seal 175 folding over itself in the gap between the base 173a of the seal and the inner surface of the liquid chamber port 159. This folding results in the wiper seal 175 flexing more than it regularly would during ordinary movement of the two components, and as such temporarily provides greater resistance to movement of the humidifier liquid chamber 151 than usual.

This increased resistance can be beneficial, as it will likely occur after the humidifier liquid chamber 151 has been connected, right before the user initially attempts to remove the humidifier liquid chamber from the recess 108. In this situation, the effect of the wiper seal 175 changing orientation would provide a temporary resistance that needs to be overcome before the humidifier liquid chamber 151 can removed. This helps prevent the humidifier liquid chamber 151 from accidentally becoming dislodged from engagement with the breathing assistance apparatus 10, but does not inhibit the removal of the humidifier liquid chamber 151 once it has begun moving relative to the housing 100.

In an advantageous configuration, the chamber connection ports 161, 163 of the breathing assistance apparatus base unit 50 would each have a single seal 173 located in a single recess, the seal having two sealing elements 175 in the form of two wiper seals. The wiper seal closer to the terminal end 163a of the gas port 163 could have a larger diameter than the other wiper seal (i.e. h1 > h2). A longer wiper seal provides a more reliable seal and accommodates variation in the inner diameter of the respective base unit connection port 157, 159 of the liquid chamber, but also requires a greater amount of travel in order to settle in the correct position. This greater amount of travel is provided by the liquid chamber 151 contacting the front wiper seal earlier when connecting the liquid chamber.

In an alternative configuration, the wiper seal closer to the terminal end 163a of the gas port 163 could have a smaller diameter than the other wiper seal (i.e. h2 > h1), but due to an internal taper of the ports 157, 159 of the humidifier liquid chamber 151, there will be a greater interference between the port 157, 159 and the wiper seal closer to the terminal end 163a of the gas port 163 than the other seal. Similarly, h1 and h2 could be equal, with greater interference between the port 157, 159 and the wiper seal closer to the terminal end 163a of the gas port 163 than the other seal, due to the internal taper of ports 157, 159. An exemplary internal taper is described below.

In another alternative configuration, the wiper seals may have the same dimensions. In another alternative configuration, the wiper seals may have three or more sealing elements 175.

In another alternative configuration, the wiper seals could be shaped to follow an internal taper of the ports 157, 159, so that there is the same interference between each wiper seal and the port 157, 159.

Alternatives to the wiper seal include using an O-ring, L-seal, or an X-ring. A wiper seal is preferred to these alternatives as it provides less resistance to the movement of the humidifier liquid chamber 151, and is easier to assemble and replace. The O-ring and X-ring have the advantage of having a higher pressure threshold; however, the pressure threshold for the wiper seal exceeds the pressures that would be used in the breathing assistance apparatus 10.

As a further alternative, one or more wiper seals could be used in addition to one of the alternative types of seals listed above or other suitable seal(s).

As shown in figures 11 and 12, the seal 173 has a further sealing element at or adjacent an opposite end of the seal 173 from the terminal end 163a of the gas port 163. In one configuration, the base 173a of the seal 173 also has an outward taper 173b leading to a radially projecting flange 177. The flange 177 is positioned at or adjacent an end of the seal 173 that is at or adjacent the proximal end 163b of the port 163 that is opposite to the terminal end 163a. The flange 177 is configured to form a seal with a component that differs from the first component (the base unit connection port 157, 159 of the humidifier liquid chamber). Therefore, the flange 177 has a configuration that differs from the wiper seals 175. In the form shown, the flange projects radially further outwardly than the wiper seals 175, so that the portion of the seal 173 with the flange has a larger diameter to connect with a second component having a larger internal diameter than the port 157, 159 of the humidifier liquid chamber. The outward taper 173b assists with guiding the different component into contact with the flange 177.

For example, the larger component may comprise part of a disinfection kit.

In an alternative configuration, the flange 177 could be an integral part of the port 161, 163 instead of the seal 173, and therefore made of hard plastic.

The terminal ends 161a, 163a of the ports 161, 163 may contact a step on the internal surface of the humidifier liquid chamber 151, thereby limiting the insertion depth of the ports 161, 163 during use. The flange 177 and taper 173b would not have any interaction with the humidifier liquid chamber 151 during use of the device, as the humidifier liquid chamber 151 does not contact the flange 177 and/or taper 173b. In an alternative configuration, the humidifier liquid chamber 151 could contact the flange 177 and/or taper 173b. This contact could result in an additional seal between the breathing assistance apparatus 10 and the humidifier liquid chamber 151.

The flange 177 and taper 173b can serve an additional purpose of providing a surface for the user to push on when connecting the removable elbow 171 to the housing 100.

The base unit 50 of the apparatus comprises a shroud 190 that cooperates with the housing 100 and the removable elbow 171. Figure 6 shows the removable elbow 171 connected to the shroud 190. As shown in figure 2 for example, the shroud 190 serves to create a uniform upper surface of the housing 100 of the apparatus 10, with the patient outlet port 30 of the removable elbow 171 protruding upwardly through the shroud 190. The shroud 190 is configured such that it is not removable from the housing 100 in normal use of the apparatus 10.

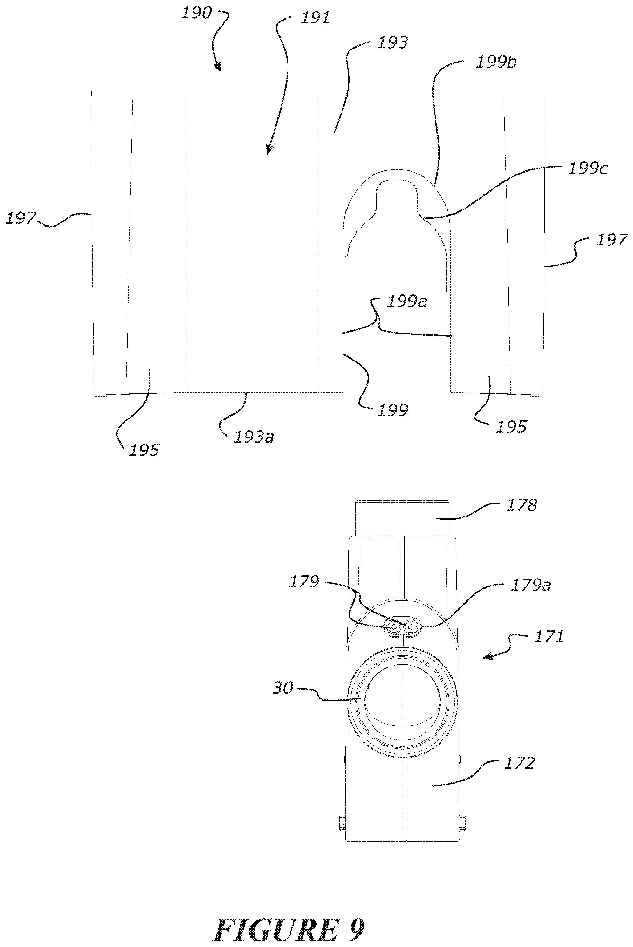

As shown in figures 6 and 9, the shroud 190 comprise a body 191 having a substantially flat horizontal upper surface 193, two contoured shoulders 195 having a substantially sinuous configuration that extend downwardly and outwardly from opposite sides of the upper surface 193, and two substantially vertical downwardly extending outer side walls 197. A recess 199 extends rearwardly into the upper surface 193 from a forward edge 193a of the upper surface 193. The recess 199 is sized and configured to receive part of the removable elbow 171, to provide an unobstructed path for the removable elbow 171 to be connected to the housing 100 of the base unit 50. In the form shown, the recess is defined by a pair of substantially parallel side walls 199a and an upper arcuate rear wall portion 199b. A contoured tapering rear wall region 199c projects into the recess from the arcuate rear wall portion, and is positioned beneath the arcuate rear wall portion 199b. The contoured tapering rear wall region is configured to receive a chimney 179a of the removable elbow.

Similarly, the removable elbow 171 has a flat horizontal tab 172 extending from the elbow that has a shape that is complementary to the shape of the recess 199 in the shroud, such that when the removable elbow 171 is assembled with the apparatus 10, the flat horizontal tab 172 is received in the recess 199 to create a uniform surface. This tab 172 can additionally provide an upper surface for the conduit 16 to contact when connecting the conduit 16 to the patient outlet port 30 of the elbow.

As shown in figure 7 and 8 for example, the flat horizontal tab 172 can have a thinned portion 172a adjacent the terminal forward end portion 172b, located between the patient outlet port 30 of the elbow and the terminal forward end portion 172b of the tab. This allows the terminal forward end portion 172b to flex vertically relative to the rest of the elbow 171.

The flat horizontal tab 172 also has engagement features comprising two protrusions 174 extending outwardly from opposite sides of the terminal forward end portion 172b of the tab. The protrusions 174 are configured to interact with engagement features comprising complementary engagement recesses 201 extending outwardly from either side wall 199a of the recess 199 of the shroud of the housing, on the underside of the shroud, as shown in figure 10. The removable elbow 171 is configured to connect to the housing by moving the removable elbow in a first direction relative to the housing (rearwards towards the housing). The removable elbow 171 is configured to disconnect from the housing by moving the removable elbow 171 in a second direction (forwards relative to the housing) that is opposite to the first direction. Due to the interaction of the engagement features, the removable elbow 171 is configured to inhibit movement of the removable elbow 171 in the second direction in the absence of actuating part of the removable elbow relative to another part of the removable elbow. That inhibits removal of the removable elbow 171 from the shroud 190 of the housing 100.

The rear portion of each protrusion 174 is designed with an angled surface 174a (figure 10). When the elbow 171 is inserted into the housing 100, this surface 174a contacts the underside of the front edge 193a of the shroud, causing the terminal end 172b of the tab to flex downwards by bending in the thinned section 172a. Once the elbow 171 has been fully inserted, the protrusion 174 will reach the complementary engagement recess 201 in the shroud. At this point the protrusion 174 will engage with the recess 201 as the tab 172 flexes back into a flat and horizontal position. When engaged, the front surface 174b of the protrusion will contact a complementary surface 201b of the engagement recess.

The front surface 174b of the protrusion is steeper, i.e. more vertical, than the rear surface 174a, such that if a user attempts to pull out the elbow 171 from the shroud 190, and thereby from the housing 100, the contact between the front surface 174b of the protrusion and the complementary surface 201b of the recess should not cause the tab to flex downwards, unless a sufficiently high force is applied to the elbow 171.

In an alternative configuration, the removable elbow 171 and shroud 190 may each have a single one of the engagement features 174, 201, rather than each having two engagement features.

In order to remove the elbow 171 from the housing 100, the user would typically first press downwards on the upper surface of the terminal forward end portion 172b of the tab 172, in order to actuate that part of the tab 172 by flexing the tab and disengage the protrusions 174 from the engagement recesses 201. Only once the tab has been flexed can a user then pull the elbow 171 out from the housing 100. One advantage of this is that it helps prevent the elbow 171 from coming loose when the humidifier liquid chamber 151 is being removed from the apparatus by pulling the humidifier liquid chamber 151 out of the recess 108.

This configuration allows a user to easily assemble the elbow 171 with the housing 100 in a single one-handed motion, but requires more complex interaction to then disassemble it. If a user does not understand how to correctly remove the elbow 171 and attempts to remove the elbow by pulling on it without actuating the tab 172, the engagement features will ultimately disengage from each other under a sufficiently high force. This will avoid damage of the removable elbow 171 and/or shroud 190.

In an alternative configuration, the engagement features may be configured such that the removable elbow 171 cannot disconnect from the shroud 190 of the housing 100 in the absence of actuating the tab 172. This could be achieved by having vertical front surfaces 174b, 201b rather than angled front surfaces.

Figure 11 schematically shows a position 176 of temperature sensor(s) such as thermistor(s) in the removable elbow 171. The thermistors are located in the rear vertical wall of the upstanding portion of the elbow, close to the curved transition region between the vertical and horizontal elbow portions. At this location, the thermistors are relatively shielded from the heat produced by the heater plate 140, allowing for more accurate estimations of the temperature of gases flowing through the removable elbow 171 to be made.

The elbow 171 has electrical connectors 179 positioned in an upstanding chimney 179a, the connectors configured to provide power from a main power board of the apparatus 10 to the heater wires 16a in the conduit 16.

As mentioned above, the shroud 190 is designed to not be removed during regular use. The shroud has features that allow it to be clipped onto a screen carrier 211, which in turn is fastened to the upper chassis 102 to become part of the housing 100. The screen carrier 211 can connect to and support a display 212. In alternative configurations, the screen carrier 211 may not be provided, and the shroud 190 may clip directly to part of the housing 100, such as an upper surface or upper chassis 102 of the housing.

The shroud 190 is configured to attach to a screen carrier (not shown) of the housing 100 via two movements; an initial movement of the shroud in a first direction followed by a subsequent movement of the shroud in a second direction that is offset from the first direction. In one configuration, the second direction is transverse to the first direction. In the form shown, the shroud 190 is configured to initially be moved in a first downward direction, followed by a movement in a second rearward direction, relative to the screen carrier and thereby the housing 100. The downward direction DD may be vertical and the rearward direction RD may be horizontal.

The shroud 190 is configured so that the shroud cannot be detached from the screen carrier 211 of the housing solely by pulling the removable elbow 171 in the second forwards direction relative to the housing 100.

Each side 197 of the shroud is shaped to be complementary to the shape of the screen carrier. The sides of the screen carrier of the housing 100 will have two forwardly directed horizontal protrusions (one per side wall) that engage with complementary rearwardly open recesses 194 in rear walls on each side of the shroud 190 as the shroud is moved in the rearward direction relative to the housing. Once the shroud 190 is connected to the screen carrier, the horizontal protrusions being received in the recesses 194 prevent vertical movement of the shroud 190.

Similarly, the screen carrier of the housing 100 will have an upstanding vertical protrusion on each side that engages with a complementary downwardly open recess 196 in the bottom of each side wall 197 of the shroud as the shroud is moved in the downward direction relative to the housing. Unlike the horizontal protrusions, the vertical protrusion is narrower than the complementary recess 196 in the shroud. This allows for a small amount of horizontal movement in the rearward direction. The vertical protrusion and recess 196 aid in aligning the shroud 190 with the screen carrier during assembly.

The shroud 190 is first placed above the screen carrier and moved in the downward direction. The shroud is then slid horizontally in the rearward direction such that the horizontal protrusions engage with the complementary recesses 194 in the shroud.

The shroud 190 and screen carrier may have a second set of forwardly extending horizontal protrusions and rearwardly open recesses 194a toward a forward end of the screen carrier and shroud 190, to further inhibit vertical movement of the shroud 190 relative to the screen carrier.

The shroud 190 and screen carrier 211 of the housing 100 may additionally have features to inhibit horizontal movement of the shroud relative to the screen carrier when they are fully engaged, such as one or more downwardly extending engagement protrusions that extend downwards from the back of the lower surface of the shroud 190. The engagement protrusions are configured to engage with complementary upwardly extending engagement protrusion(s) extending from an upper surface of the screen carrier.

The removable elbow 171 is removable from the housing 100 when the shroud 190 is attached to the housing.

In an alternative configuration, one or more of the engagement protrusions can be replaced with an engagement recess. One side of the engagement recess would have a surface that is complementary to the second side of the engagement protrusion in order to inhibit disassembly in a similar way to the above configuration. In a further configuration, one or more of the engagement protrusions could be replaced with a combination of an engagement protrusion and engagement recess, such that a complementary engagement protrusion engages over the engagement protrusion and within the engagement recess.

A similar shroud 190 and screen carrier 211 and/or housing 100 engagement configuration could be used where the shroud 190 acts as a cover for part of the housing or another component, but where the component that is covered is not removable.

Referring to figure 7 and 8, the removable elbow 171 also includes an electrical connection. The elbow 171 has an inlet for pneumatically connecting to a first accessory for the breathing assistance apparatus 10, such as the humidifier liquid chamber 151, an outlet for pneumatically and optionally electrically connecting to a second accessory for the breathing assistance apparatus, such as the patient breathing conduit 16, and a printed circuit board (PCB) electrical connector 178 for electrically connecting to the breathing assistance apparatus 10 to form an electrical connection with an electrical component in the housing. The electrical connection provides an electrical link between the base unit 50 and the temperature sensors 176 embedded in the elbow, as well as between the base unit 50 and the conduit 16 (when the conduit has one or more sensors and/or a heating element), via an electrical interconnecting assembly 221 in the housing which will be described below. The PCB electrical connector 178 electrically connects to the electrical interconnecting assembly 221 when the removable elbow 171 is connected to the housing 100.

The pneumatic inlet connection of the removable elbow 171 is provided by the humidified gas inlet port 163, the pneumatic outlet connection is provided by the patient outlet port 30, and the outlet electrical connection is provided by connectors 179 (figure 9) that are provided in the chimney 179a that extends upwardly parallel to an axis of the port 30.

The humidified gas inlet port 163 and the patient outlet port 30 are in fluid communication with each other via a gas flow path in the removable elbow. Electrical connectors 178, 179 of the removable elbow are pneumatically isolated from the gas flow path between the humidified gas inlet port 163 and the patient outlet port 30, via at least one wall of the removable elbow. For example, the body of the removable elbow may be injection moulded plastic material, with isolated regions provided for the electrical connectors that are separate and isolated from the gas flow path.

Because the portion of the removable elbow, namely PCB electrical connector 178, that is configured to form the electrical connection with the electrical component in the housing (the electrical interconnecting assembly 221) is pneumatically isolated from the gas flow path of the removable elbow, coupling the removable component to the housing solely provides an electrical connection between the PCB electrical connector 178 and the electrical interconnecting assembly 221. It does not form a direct pneumatic connection between the gas flow path in the removable elbow 171 and the housing 100. Instead, the gas flow path in the removable elbow provides a pneumatic connection between the first accessory (the humidifier liquid chamber 151) and the second accessory (the patent breathing conduit 16).

In the configuration shown, the PCB electrical connector 178 is partly housed in a housing 178a that is integrally formed with the elbow. The PCB electrical connector projects rearwardly from the housing 178a to insert into an electrical connector on the base unit 50 in a horizontal direction (i.e. the same rearward insertion direction CID in which the humidifier liquid chamber 151 connects to the base unit 50 and the same rearward direction RD in which the shroud 190 connects to the screen carrier 211). As such, the removable elbow 171 can be horizontally connected to the apparatus in the rearward direction RD, with the humidifier liquid chamber 151 being horizontally connected to the two chamber connection ports 161, 163 after that. Alternatively, the humidifier liquid chamber 151 can initially be connected to the removable elbow 171, with the assembled humidifier liquid chamber 151 and elbow 171 then being connected to the base unit 50 together by moving them in the rearward direction together.

This allows the components to be assembled and disassembled in multiple ways. Specifically, the elbow 171 will make three connections during assembly. These three connections are the connection between the elbow 171 and the patient breathing conduit 16, the connection between the elbow 171 and the humidifier liquid chamber 151, and the connection between the elbow 171 and the housing 100. Each of these three connections can be made in any order. Similarly, when disassembling the components, these three connections can be broken in any order. This is advantageous as different orders of assembly and disassembly may be preferred in different scenarios.

For example, the removable elbow 171 may be assembled with the housing 100 initially as the removable elbow may have come assembled with the housing 100, with the humidifier liquid chamber 151 and the conduit 16 not being attached until the apparatus 10 is to be used. As the removable elbow 171 is removed much less frequently than the humidifier liquid chamber 151 and/or conduit 16, this will be the most common order of assembly. Additionally, in a hospital setting, the humidifier liquid chamber 151 and conduit 16 would be replaced between each patient, while the removable elbow 171 may only be cleaned/disinfected between uses. As such, the removable elbow 171 can be assembled with the housing 100 after being cleaned, with the humidifier liquid chamber 151 and conduit 16 again only being connected when a patient needs to use the apparatus 10.

The humidifier liquid chamber 151 and conduit 16 may also be removed prior to removing the elbow 171 after the apparatus 10 has been used in order to first throw these components away, with the removable elbow 171 then remaining with the apparatus 10 until it is to be cleaned. The removable elbow 171 may be cleaned while connected to the housing 100 through use of a disinfection kit as described above.

The conduit 16 and/or humidifier liquid chamber 151 may be preassembled with the removable elbow 171 after having cleaned the elbow 171, thereby allowing all three components to be able to quickly connect to the housing 100 in one motion when required.

The removable elbow 171 may be disconnected from the housing 100 with the conduit 16 and humidifier liquid chamber 151 still attached, which may be useful, for example, in situations in which the patient has a particularly infectious disease. In these situations, it may be desirable to disconnect the removable elbow 171 in this way, such that the circuit is largely still contained and can easily be thrown away in its entirety.

Connecting and disconnecting the preassembled removable elbow 171, conduit 16 and humidifier liquid chamber 151 to/from the housing 100 is facilitated by the removable elbow 171 and humidifier liquid chamber 151 connecting in the same direction (i.e. rearwardly in a horizontal direction), as well as the shroud 190 not needing to be attached over the removable elbow 171 after the removable elbow 171 has been inserted.

The PCB electrical connector 178 of the removable elbow 171 inserts into an electrical interconnecting assembly of the base unit 50. The interconnecting assembly may be made up of multiple components, such as a socket, a PCB, and an overmould. The socket defines a receptacle to receive the PCB electrical connector of the removable elbow.

An alternative form breathing assistance apparatus 10 may be a standalone humidifier apparatus comprising a base unit 50 defining a main housing and a humidifier 12.

The standalone humidifier apparatus can deliver heated and humidified gases for various medical procedures, including respiratory therapy, laparoscopy, and the like. These apparatuses can be configured to control temperature and/or humidity. The apparatuses can also include medical circuits comprising various components that can be used to transport heated and/or humidified gases to and/or from patients. For example, in some breathing circuits, gases inhaled by a patient are delivered from a heater-humidifier through an inspiratory tube or conduit. As another example, tubes can deliver humidified gas (commonly CO₂) into the abdominal cavity in insufflation circuits. This can help prevent desiccation or 'drying out' of the patient's internal organs, and can decrease the amount of time needed for recovery from surgery. Heater wires may extend inside of at least a portion of the tubing forming the circuit to prevent or at least reduce the likelihood of the formation of significant condensation.

A standalone humidifier apparatus would typically include a base unit 50 and a humidifier liquid chamber 151. The base unit 50 can comprise a heater plate 140. The humidifier liquid chamber 151 can be configured to hold a volume of a liquid, such as water. The heater plate can be configured to heat the volume of liquid held within the humidifier liquid chamber 151 to produce vapour.

The humidifier liquid chamber 151 is removable from the base unit to allow the humidifier liquid chamber to be more readily sterilized or disposed, or to re-fill the humidifier liquid chamber with liquid. The body of the humidifier liquid chamber 151 can be formed from a non-conductive glass or plastics material but the humidifier liquid chamber can also include conductive components. For instance, the humidifier liquid chamber can include a highly heat-conductive base (for example, an aluminum base) contacting or associated with the heater plate on the heater base.

The base unit can also include electronic controls such as a master controller. In response to user-set humidity or temperature values input via a user interface and other inputs, the master controller determines when (or to what level) to energize the heater plate 140 to heat the liquid within the humidifier liquid chamber 151.

The standalone humidifier apparatus can include a flow generator to deliver gases to the humidifier liquid chamber. In some configurations, the flow generator can comprise a ventilator, blower, or any other suitable source of pressurized gases suitable for breathing or use in medical procedures. The flow generator may be positioned in the base unit 50.

Alternatively, the standalone humidifier apparatus may comprise just the base unit 50 and the humidifier liquid chamber 151, and may be used with a separate or remote flow generator. The base unit 50 may be configured to fluidly connect to the separate or remote flow generator.

Therefore, the flow generator that is used with a standalone humidifier apparatus may be a wall gases source, ventilator, blower, or gas tank for example.

A standalone humidifier apparatus can be used with breathing therapies, positive pressure apparatus, noninvasive ventilation, surgical procedures including but not limited to laparoscopy, and the like. Desirably, the humidifier apparatus can be adapted to supply humidity or vapour to a supply of gases. The humidifier apparatus can be used with continuous, variable, or bi-level PAP systems or other form of respiratory therapy. In some configurations, the humidifier apparatus can be integrated into a system that delivers any such types of therapy.

An exemplary standalone humidifier apparatus is described in WO 2015/038013.

The standalone humidifier apparatus may have any one or more of the features described or shown herein, to enable interaction with the humidifier liquid chamber 151, 151' and/or manifold 1701.

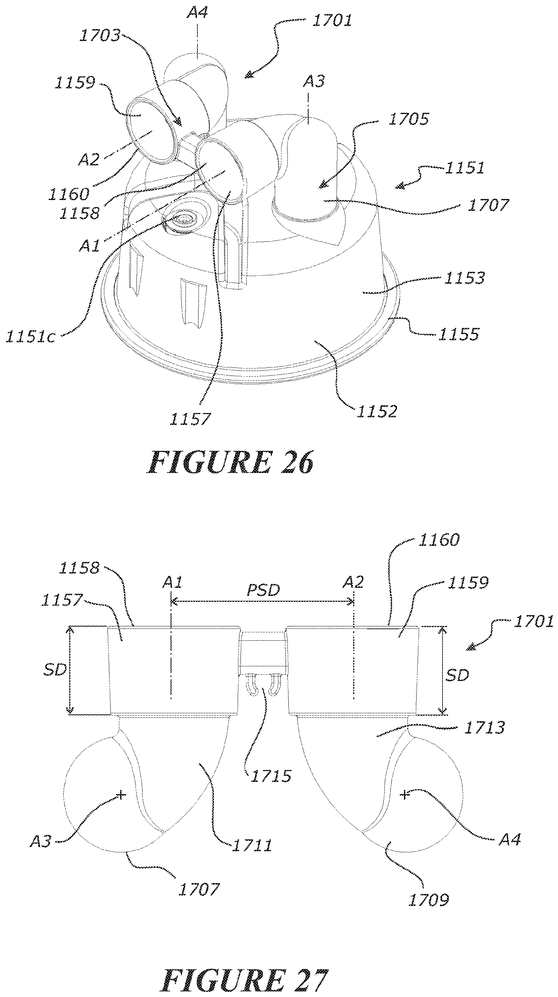

Figures 3, 13-22 and 23 to 25 show features of the humidifier liquid chamber 151 for use with the breathing assistance apparatus 10. The features that are shown in the figures and described herein for humidifier liquid chamber 151 can be implemented in the humidifier liquid chamber 151 or in a humidifier liquid chamber manifold 1701 for use with an alternative humidifier liquid chamber 1151, such as shown in Figures 27 to 30. Like numbers are used to indicate like parts for the manifold 1701, with the addition of 1000.

The humidifier liquid chamber 151 has first and second base unit connection ports defined by the gas inlet port 157 and gas outlet port 159. The base unit connection ports 157, 159 are for connecting to the breathing assistance apparatus base unit 50. The gas outlet port 161 and humidified gas inlet port 163 of the breathing assistance apparatus base unit 50 define first and second chamber connection ports of the breathing assistance apparatus base unit 50. The first and second base unit connection ports 157, 159 are configured to connect to the first and second chamber connection ports 161, 163 respectively.

In use, the humidifier liquid chamber 151 receives a flow of gas from the gas outlet port 161, humidifies the flow of gas, then delivers the gas to the humidified gas inlet port 163.

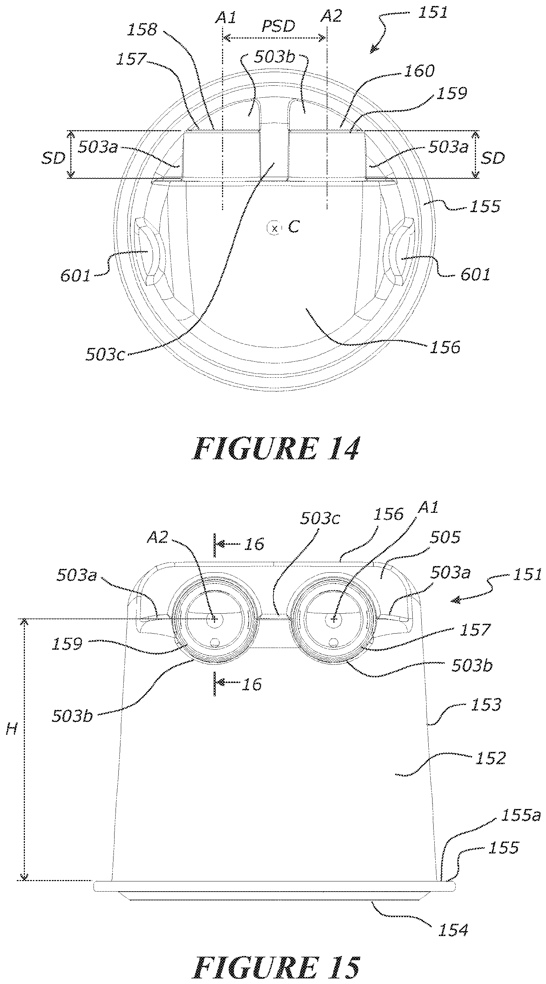

The first and second base unit connection ports 157, 159 define respective axes A1, A2 (Figure 14). The base unit connection ports are substantially parallel to each other and are separated by a port separation distance PSD between the axes A1, A2.

The first and second base unit connection ports 157, 159 are configured to be in a substantially horizontal orientation when the humidifier liquid chamber is in use.

At least one of, and optionally both of, the base unit connection ports 157, 159 has a sealing depth SD defined by a portion of the port 157, 159 that is configured to overlap with a complementary chamber connection port 161, 163 of the breathing assistance apparatus base unit 50. The at least one of the base unit connection ports 157, 159 is configured to interact with the seal 173 of at least one of the chamber connection ports 161, 163.

A ratio of sealing depth SD to port separation distance PSD is designed for accurate chamber connection port 161, 163 connection and sealing. As discussed above, a complete seal between the base unit connection ports 157, 159 and the chamber connection ports 161, 163 is not required; some leakage may be accommodated between those components while still providing sufficient gas flow to a user.

In one exemplary configuration, the port separation distance PSD of the chamber connection ports 161, 163 of the breathing assistance apparatus base unit 50 is 40mm and there are two seal elements 175 on each seal 173. The primary seal is 6mm from the humidifier liquid chamber end 161a, 163a of the port 161, 163 and the secondary seal is 12mm from the humidifier liquid chamber end 161a, 163a of the port 161, 163.

A ratio of the sealing depth SD to port separation distance PSD of the base unit connection ports 157, 159 is more than about 0.25, more than about 0.25 and up to about 0.7, between about 0.3 and about 0.7, between about 0.3 and about 0.6, between about 0.4 and 0.5, between about 0.45 and 0.5, or about 0.475. The ratio of sealing depth SD to port separation distance PSD of the base unit connection ports may be about 0.25, about 0.275, about 0.3, about 0.325, about 0.35, about 0.375, about 0.4, about 0.425, about 0.45, about 0.475, about 0.5, about 0.525, about 0.55, about 0.575, about 0.6, about 0.625, about 0.65, about 0.675, about 0.7, or any value or range between those values.

Increasing this ratio leads to easier visual alignment for the user, owing to concentric connection between the ports 157, 159, 161, 163, as well as increasing the likelihood of a successful seal. However, increasing the ratio beyond 0.7 could impact the ability of the humidifier liquid chamber to appropriately humidify the flow of gas. In one scenario the extension flow tubes 301, explained in the following section, would interfere with the roof of the humidifier liquid chamber and may need to be redesigned or the humidifier liquid chamber itself becomes unlikely to fit within the designated space and engage properly with the breathing assistance apparatus base unit 50.

The sealing depth SD is more than about 10 mm, more than about 10 mm and up to about 16mm, more than about 12 mm, or more than about 12 mm and up to about 16 mm. The sealing depth SD may be about 10 mm, about 11 mm, about 12 mm, about 13 mm, about 14 mm, about 15 mm, about 16 mm, or any value or range between those values.

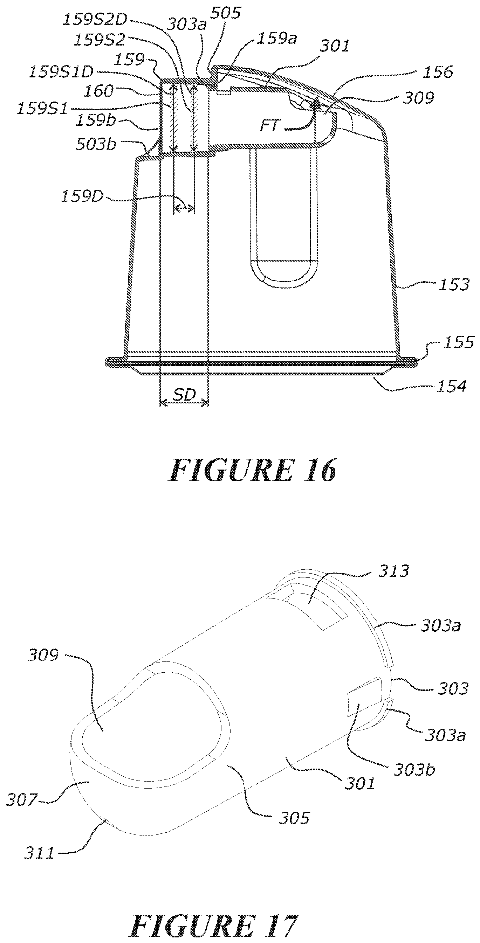

The sealing depth SD is shown in Figure 16, and extends from the exterior face 160 of the base unit connection port 159 to the front of the flange 303a (and will correspond on the other base unit connection port 157). The sealing depth represents the maximum extent of overlap between the ports 157, 159, 161, 163.

The port separation distance PSD is between about 35 mm and about 45 mm, between about 38 mm and about 42 mm, or is about 40 mm +/- 0.6mm. The port separation distance PSD may be about 35 mm, about 36 mm, about 37 mm, about 38 mm, about 39 mm, about 40 mm, about 41 mm, about 42 mm, or any value or range between those values.

With reference to Figure 15, a distance H between a central axis A1, A2 of the base unit connection ports 157, 159 and a lower surface of the body 152 of the humidifier liquid chamber 151 is at least about 85 mm, or at least about 86 mm, or at least about 87 mm, or at least about 88 mm. That is, the central axis A1, A2 of each base unit connection port is at least about 85 mm, or at least about 86 mm, or at least about 87 mm, or at least about 88 mm above the lower surface of the body of the humidifier liquid chamber.

In some configurations, the lower surface of the body 152 of the humidifier liquid chamber is defined by the upper edge 155a of the lower flange 155 of the body 152 of the humidifier liquid chamber.

The lower surface of the body 152, i.e. the upper edge of the lower flange 155, is configured to engage with one or more retaining features in the breathing assistance apparatus base unit to assist with retaining the liquid chamber 151 in engagement with the breathing assistance apparatus base unit.

For example, the retaining feature(s) may be provided in the recess 108 to assist with retaining the humidifier liquid chamber 151 in position in the recess 108 and the base unit connection ports 157, 159 in engagement with the chamber connection ports 161, 163. The distance between the retaining feature(s) in the recess 108 and the centres of the chamber connection ports 161, 163 will correspond to the distance H between the central axes A1, A2 of the base unit connection ports 157, 159 and the lower surface of the body 152 of the humidifier liquid chamber 151.

When the heater plate 140 is resiliently mounted, the heater plate can accommodate variations in distance between the base flange 155 and the bottom of the base 154 of the humidifier liquid chamber.

The at least one of the base unit connection ports 157, 159 is configured to seal with at least two seals 175 on the complementary chamber connection port 161, 163.

In some configurations, both of the base unit connection ports 157, 159 are configured to overlap with the complementary chamber connection ports 161, 163,

In some configurations, both of the base unit connection ports 157, 159 are configured to interact with a seal 173 of the chamber connection ports 161, 163.

In some configurations, each of the base unit connection ports 157, 159 is configured to seal with at least two seals 175 on the complementary chamber connection ports 161, 163.

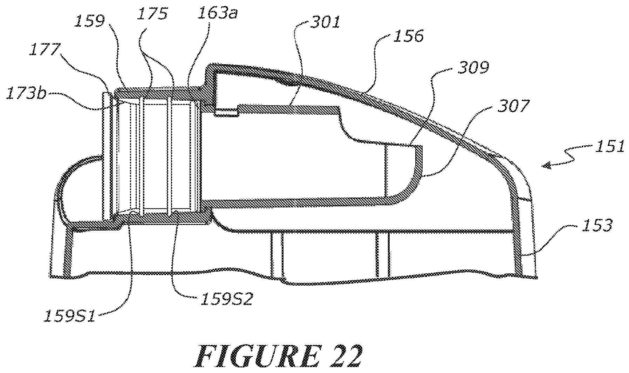

With reference to Figures 16 and 22, each of the base unit connection ports 157, 159 of the humidifier liquid chamber 151 comprises at least two sealing portions 15951, 159S2 configured to form at least two seals with a portion of the breathing assistance apparatus base unit 50 when connected to the breathing assistance apparatus base unit 50.

In the configuration shown, each base unit connection port 157, 159 has two sealing portions 15951, 159S2 configured to form at least two seals with the two sealing elements 175 on the complementary chamber connection ports 161, 163. Alternatively, each base unit connection port 157, 159 could have three or more sealing portions configured to form three or more seals with three or more sealing elements.

With reference to Figure 16, the two sealing portions 15951, 159S2 are separated by a distance 159D of about 5 mm to about 7 mm in an axial direction of the base unit connection ports. In alternative configurations, the two sealing portions 15951, 159S2 could be separated by a distance 159D of about 5.5 mm to about 6.5 mm, or by a distance of about 6 mm.

As outlined above, each base unit connection port 157, 159 of the humidifier liquid chamber 151 may comprise an internal taper such that the port narrows from a terminal end 159b of the base unit connection port towards an opposite end of the base unit connection port. The internal taper could have any suitable angle, such as between about 2 degrees and about 10 degrees, or between about 2 degrees and about 8 degrees, or between about 2 degrees and about 6 degrees, or between about 3 degrees and about 5 degrees, or about 4 degrees for example.

In one configuration, an inner diameter 159S1D of a first one of the sealing portions proximal to the terminal end 159b of the base unit connection port 157, 159 is about 0 mm to about 0.6 mm greater than an inner diameter 15952D of a second one of the sealing portions distal from the terminal end 159b of the base unit connection port. That is, in some configurations, the first sealing portion 159S1 may be the same size as the second sealing portion 159S2, or may be larger. In some configurations, the inner diameter 15951D of the first sealing portion 159S1 may be more than about 0 mm and up to about 0.6 mm greater than the inner diameter 159S2D of the second sealing portion 15952.

In one configuration, the inner diameter 15951D of the first one of the sealing portions is less than about 25.6 mm, or less than about 25.4 mm, or less than about 25.2 mm. In one configuration, the inner diameter 159S2D of the second one of the sealing portions is less than about 25.4 mm, or less than about 25.2 mm, or less than about 25.0 mm.

The inner surface of each base unit connection port 157, 159 may be substantially continuous between and including the first and second sealing portions 15951, 15952. Alternatively, the inner surface may be discontinuous. For example, a step could be provided between the first sealing portion 15951 and the second sealing portion 15952.

The overlapping length of the base unit connection ports 157, 159 and the chamber connection ports 161, 163 is sufficient to provide improved protection against liquid ingress.

The base unit connection ports 157, 159 each have an exterior face 158, 160. The exterior faces 158, 160 are front faces of the base unit connection ports 157, 159 and are at an end of the base unit connection ports that are distal from the rear ends of the base unit connection ports that connect to the humidifier liquid chamber body 152.

The exterior faces 158, 160 are substantially planar and are substantially perpendicular to the axes A1, A2 of the base unit connection ports 157, 159.

With reference to Figure 16 and 17, the humidifier liquid chamber 151 comprises extension flow tubes 301 extending into the interior of the liquid chamber from the first and second base unit connection ports 157, 159.

The extension flow tubes 301 may be inserted into the first and second base unit connection ports 157, 159 or alternatively may be integrally formed with the humidifier liquid chamber 151.

Each flow tube 301 comprises a tubular finger-shaped member having a front end 303 and an elongate body 305 that terminates at a rear end 307. The rear end 307 of the flow tube is outwardly convex and inwardly concave, to provide a smooth flow transition FT from a generally horizonal flow direction to a generally vertical flow direction.

The flow tube 301 can be inserted into the respective base unit connection port 157, 159 from the front end 158, 160 of the port 157, 159. The front end 303 of the flow tube 301 comprises one or more flanges 303a (and in the form shown, two flanges 303a) that define a rearward movement stop that engages against a shoulder 159a at the interface between the port 157, 159 and the humidifier liquid chamber body 152. The shoulder is formed by vertical wall 505.

The front end 303 of the flow tube 301 also includes one or more engagement features 303b to inhibit removal of the flow tube 301 from the base unit connection port 157, 159 once the flow tube 301 is engaged with the base unit connection port 157, 159.

The engagement feature(s) 303b is/are spaced from the flange(s) 303a.

In the form shown, the engagement feature(s) 303b comprise(s) a tapered wedge member having a large dimension adjacent the flange 303(a) and a small dimension spaced further from the flange 303(a). This configuration allows the flow tube to be readily inserted into the base unit connection port 157, 159, with part of the shoulder engaging against the large dimension of the wedge member, and inhibits disengagement of the flow tube from the shoulder base unit connection port. Other types of engagement features could be used, such as radially extending projections.

The flow tube 301 comprises an opening 309 at or adjacent the rear end 307 of the flow tube that is distal from the respective base unit connection port 157, 159. The opening 309 is configured to direct flow and enable the generally vertical flow to exit the flow tube, and is spaced from a wall of the humidifier liquid chamber.

The opening 309 is shaped to help reduce the potential for splashing or liquid entrainment to occur.

In the form shown in Figure 16, the opening 309 faces a roof 156 of the humidifier liquid chamber 151, and is spaced from the roof. Alternatively, the opening 309 could face a side wall 153 of the humidifier liquid chamber 151 and be spaced from the side wall. Alternatively, the opening 309 could be angled to face both the roof 156 and the side wall 153 of the humidifier liquid chamber, and could be spaced from the roof and the side wall.

The flow tube 301 comprises a liquid drainage aperture 311 at or adjacent the rear end 307 of the flow tube that is distal from the respective base unit connection port. The drainage aperture enables liquid to drain back into the humidifier liquid chamber after filling the humidifier liquid chamber by one of the ports 157, 159, or that has built up from condensation.

The flow tube 301 comprises a gas bleed aperture 313 in an upper surface of the flow tube. In the form shown, the gas bleed aperture 313 is rectangular in shape and is positioned adjacent the front end 303 of the flow tube. The gas bleed aperture 313 could alternatively be positioned elsewhere in the flow tube, such as closer to the rear end 307. The gas bleed aperture 313 could have different shapes, such as square or circular for example.

The gas bleed aperture 313 allows the humidifier liquid chamber 151 to be filled to the intended maximum liquid fill level when the humidifier liquid chamber is oriented on its rear and liquid is poured into the humidifier liquid chamber via the ports 157, 159. In such an orientation, the gas bleed aperture 313 allows air in the humidifier liquid chamber 151 to be displaced by the liquid entering the humidifier liquid chamber and filling it up. Without this aperture when the liquid is filled to a certain level, any air in the humidifier liquid chamber can no longer be displaced as the air passage out of the humidifier liquid chamber would be blocked.

Alternatively, a gas bleed aperture 313 can be absent and a funnel can be used to fill up the liquid chamber.

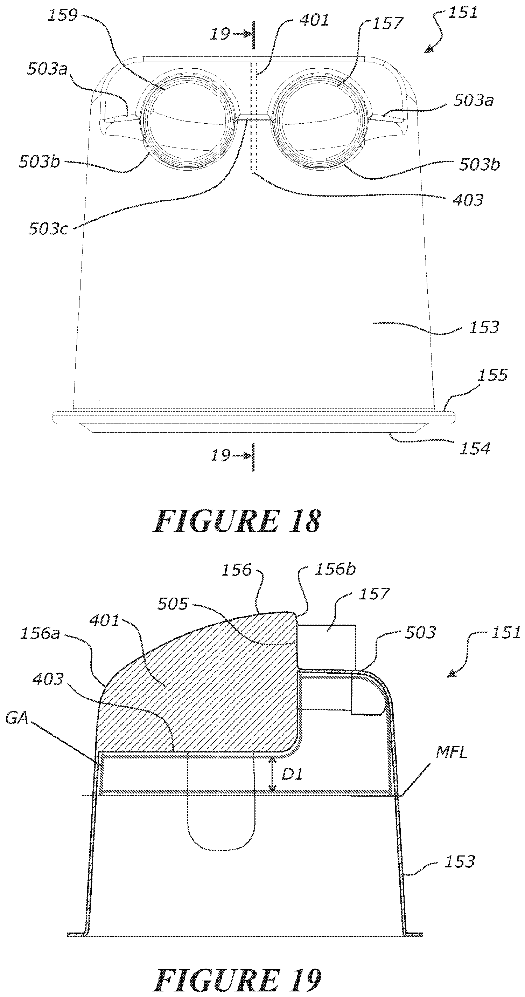

With reference to Figures 18, 19, and 23, the humidifier liquid chamber 151 comprises a baffle 401 in the interior of the humidifier liquid chamber. The baffle 401 is located between the base unit connection ports 157, 159 and between the openings 309 of the flow tubes 301.

The baffle 401 is in the form of a wall. The wall is oriented substantially vertically. The wall has relatively large vertical and forward-rearward dimensions, and a relatively small transverse dimension.

The baffle 401 could be a one-piece component, or could be formed from multiple elements.

The baffle 401 extends downward from the roof 156 of the humidifier liquid chamber 151 and terminates above a maximum liquid fill level MFL of the humidifier liquid chamber.

As used herein, 'maximum liquid fill level' means the maximum level to which the humidifier liquid chamber 151 can be filled with liquid and/or the maximum level to which a user is intended to fill the humidifier liquid chamber 151 with liquid.

A lower edge 403 of the baffle 401 is positioned above a surface of the liquid in the humidifier liquid chamber when the liquid chamber is filled to the maximum liquid fill level for use of the liquid chamber.

As the flow of gas enters the humidifier liquid chamber 151 it impacts the roof 156 of the humidifier liquid chamber and is directed downwards towards the liquid surface. The baffle 401 minimises the ability for the flow of gas to exit the humidifier liquid chamber 151 before becoming appropriately humidified by creating a tortuous flow path between the inlet and outlet extension flow tubes 301. The baffle 401 increases the minimum residence time for gases within the humidifier liquid chamber. The baffle 401 terminates above the water surface and at the vertical face 505. This leaves an area for the flow of gas, enclosed by the base 503 of the step, baffle 401, chamber wall 153 and liquid surface MFL; highlighted as region GA in Figure 19. When the humidifier liquid chamber 151 is filled with liquid to the maximum liquid fill level MFL (not shown to scale in Figure 19), the liquid surface is a suitable distance D1 below the bottom edge 403 of the baffle 401.

The distance D1 may be between about 5 mm and about 15mm. The distance may be between about 6 mm and about 14 mm, between about 7 mm and about 13 mm, between about 8 mm and about 12 mm, between about 9 mm and about 11 mm, about 5mm, about 6mm, about 7 mm, about 8 mm, about 9 mm, about 10 mm, about 11 mm, about 12 mm, about 13 mm, about 14 mm, about 15 mm, or any value or range between those values.

The roof 156 of the humidifier liquid chamber is sloped so as to be substantially non-horizontal in use. In the form shown, the roof 156 slopes rearwardly, so that the roof is lower at its rear edge 156a than at its forward edge 156b (Figure 19). The roof 156 may be substantially arcuate as shown.

At least one of, and in some configurations all of, the roof 156, the baffle 401, and wall(s) 153 of the humidifier liquid chamber are configured to direct gases exiting the opening 309 of the flow tube associated with the first base unit connection port 157 toward and over the surface of liquid in the humidifier liquid chamber.

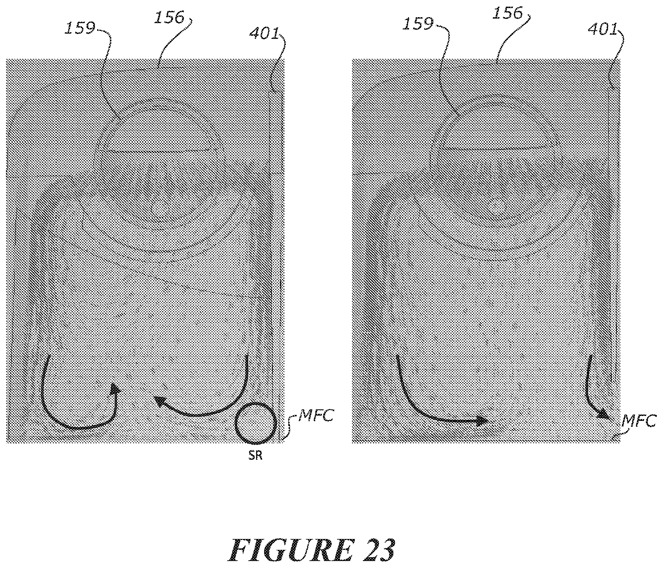

By having a space between the maximum liquid fill level MFL and the lower edge 403 of the baffle 401, there is substantially no deadspace (i.e. stagnation region) in the humidifier liquid chamber in use. That is, there are substantially no spots in the interior of the humidifier liquid chamber above the liquid level, at which the gas flow is stagnant or still. This is shown in Figure 23.

Figure 23 shows the effect of baffle height for a baffle spaced above the maximum liquid fill level (right side) versus a baffle that is not spaced above a maximum liquid fill level (left). The curved arrows are overlaid for emphasis on a plot of the velocity vectors for the flow of gases. A stagnation region SR is evident in the inlet side of the humidifier liquid chamber in the left side plot, and the reduction of stagnation is evident for the inlet side of the humidifier liquid chamber in the right side plot.

Increasing the distance from the openings 309 of the extension flow tubes 301 to the lower edge 403 of the baffle 401 (by having upwardly oriented openings 309), provides increased relative humidity of the outlet gases.

In an alternative configuration, the baffle 401 extends downward from a roof 156 of the humidifier liquid chamber and terminates at or below the intended maximum liquid fill level MFL of the humidifier liquid chamber. In that configuration, the lower edge 403 of the baffle 401 is positioned at or below a surface of the liquid in the humidifier liquid chamber when the humidifier liquid chamber is filled with an intended liquid level for use of the humidifier liquid chamber.

With reference to Figures 13, 15, and 19, the humidifier liquid chamber 151 comprises a step 501. The step 501 is located in the upper front region of the humidifier liquid chamber and forms a recess for the first and second base unit connection ports 157, 159.

A base 503 of the step is located at least partly below the first and second base unit connection ports 157, 159. In some configurations, the entire base 503 is located below the first and second base unit connection ports 157, 159.

In the form shown, the base 503 of the step has a staggered shape so that outer regions 503a of the base adjacent the sides of the humidifier liquid chamber 151 are relatively high, intermediate regions 503b of the base corresponding to the position of the first and second base unit connection ports 157, 159 are relatively low, and a central region 503c between the base unit connection ports is relatively high. In the form shown, the outer regions 503a and central region 503c are positioned about half way up the base unit connection ports 157, 159, and the intermediate regions are positioned below the base unit connection ports 157, 159. However, the heights of those regions relative to the connection ports could differ.

The intermediate regions 503b are arcuate in front view with a curvature substantially corresponding to the curvature of the base unit connection ports 157, 159. The intermediate regions 503b extend forwardly beyond the exterior faces 158, 160 of the first and second base unit connection ports 157, 159, and may contact rear portions of the chamber connection ports 161, 163 when the humidifier liquid chamber 151 is engaged with the breathing assistance apparatus base unit 50.

The rear of the base 503 connects to a substantially vertical face 505 of the step. The substantially vertical face 505 extends upwardly from the base 503 and connects to the roof 156. The base unit connection ports 157, 159 protrude forwardly from the substantially vertical face 505.

The baffle 401 extends from a rear of the liquid chamber body 152 to the substantially vertical face 505 of the step. Having the baffle 401 starting from the rear of the humidifier liquid chamber body 152 prevents gases that exit from the gas inlet port 157, to directly flow around the baffle into the gas outlet port 159, without adequately picking up moisture from the water.

As shown in Figure 19, the baffle 401 extends downwardly beyond the base 503 of the step, so that the lower edge 403 of the baffle is positioned lower than the base 503 of the step.

A shoulder is located between the first and second base unit connection ports 157, 159, such that the first and second base unit connection ports 157, 159 are located on either side of the shoulder. The shoulder is formed by the central region 503c of the base 503 of the step 501.

The shoulder 503c extends forwardly beyond the exterior faces 158, 160 of the first and second base unit connection ports 157, 159. The shoulder 503c helps with physical and visual alignment of the humidifier liquid chamber 151 with the apparatus. The shoulder 503c aligns with the space between the chamber connection ports 161, 163 of the apparatus.

The shoulder 503c may have an arcuate upper/forward surface as shown.

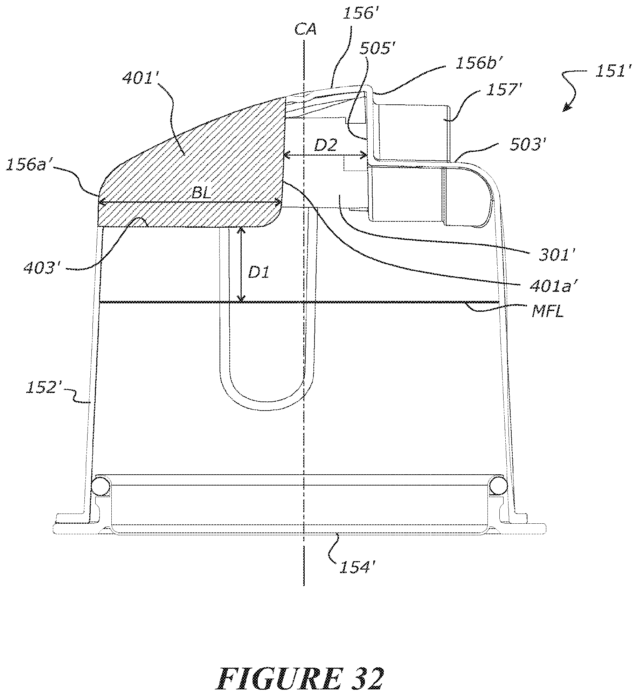

In an alternative configuration of the humidifier liquid chamber 151' shown in Figure 32, the baffle 401' extends from a rear of the humidifier liquid chamber body 152' towards the substantially vertical face 505' of the step, and terminates proximal to a central axis CA of the humidifier liquid chamber 151'.

In some configurations, a spacing or distance D2 between a forward edge 401a' of the baffle and the substantially vertical face 505' of the step is at least about 20 mm, or at least about 21 mm, or at least about 22 mm, or at least about 23 mm.

In some configurations, the length BL of the baffle from the rear edge of the baffle adjacent the rear edge 156a' of the roof to the forward edge 401a of the baffle is between about 40 mm and about 50 mm. In some configurations, the length BL is between about 41 mm and about 49 mm, or between about 42 mm and about 48 mm, or between about 43 mm and about 47 mm, or between about 44 mm and about 46 mm, or about 45 mm.

Providing a spacing or distance between the forward edge 401a' of the baffle and the substantially vertical face 505' of the step may assist with avoiding splashing of liquid out of the outlet port 157' when high flow rates are used.

Other than these recited features, the features and functioning of the baffle 401' and related features are the same as for the baffle 401 and related features of the humidifier liquid chamber 151, and like reference numbers indicate like parts with the addition of a prime (').

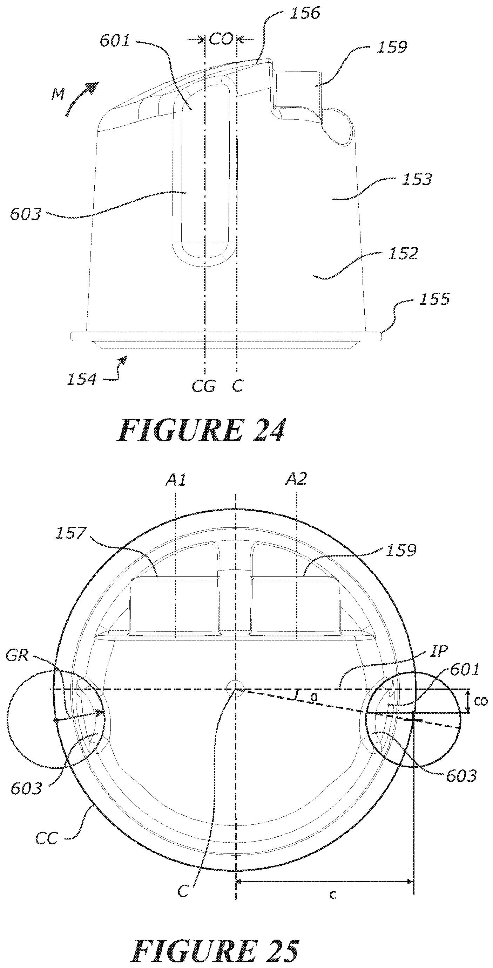

Referring to Figures 24 and 25, the humidifier liquid chamber comprises a base 154, a top formed by the roof 156, and one or more side walls 153.

The humidifier liquid chamber 151 has a rounded exterior shape with generally smooth sides. This could make it difficult for the user to hold during connection or disconnection with the breathing assistance apparatus base unit 50, especially during connection when the humidifier liquid chamber is heavier due to being filled with liquid.

To connect the humidifier liquid chamber 151 to the breathing assistance apparatus base unit 50, the guard 160 is flexed downwardly to enable the humidifier liquid chamber 151 to slide rearwardly CID toward the breathing assistance apparatus base unit 50. The guard 160 protects the user from touching the heater plate 140 on the breathing assistance apparatus. Owing to the weight and geometry of the humidifier liquid chamber 151 and the required orientation and force for connection, it is most likely the humidifier liquid chamber 151 will be grasped by the sides.

For this reason, at least two grips 601 are located in the side wall(s) of the humidifier liquid chamber 151 to enable grasping by a user. The grips 601 are located at an angle α relative to a plane IP that extends through a centre C of the humidifier liquid chamber transversely to the axes A1, A2 of the inlet port 157 and the outlet port 159. The plane IP is an imaginary plane that extends across the humidifier liquid chamber 151 through the centre C of the humidifier liquid chamber.

The angle α is between about 0 degrees and about 40 degrees relative to the plane IP. In some configurations, the angle α is between about 10 degrees and about 40 degrees relative to the plane IP. In some configurations, the angle α is between about 10 degrees and about 30 degrees, between about 10 degrees and about 20 degrees, or between about 10 degrees and about 15 degrees relative to the plane IP. In some configurations, the angle α is between about 11 degrees and about 14 degrees, between about 12 degrees and about 13 degrees, or about 12.5 degrees relative to the plane IP. The angle α may be about 10 degrees, about 10.5 degrees, about 11 degrees, about 11.5 degrees, about 12 degrees, about 12.5 degrees, about 13 degrees, about 13.5 degrees, about 14 degrees, about 14.5 degrees, about 15 degrees, about 15.5 degrees, about 16 degrees, about 16.5 degrees, about 17 degrees, about 17.5 degrees, about 18 degrees, about 18.5 degrees, about 19 degrees, about 19.5 degrees, about 20 degrees, about 20.5 degrees, about 21 degrees, about 21.5 degrees, about 22 degrees, about 22.5 degrees, about 23 degrees, about 23.5 degrees, about 24 degrees, about 24.5 degrees, about 25 degrees, about 25.5 degrees, about 26 degrees, about 26.5 degrees, about 27 degrees, about 27.5 degrees, about 28 degrees, about 28.5 degrees, about 29 degrees, about 29.5 degrees, about 30 degrees, about 30.5 degrees, about 31 degrees, about 31.5 degrees, about 32 degrees, about 32.5 degrees, about 33 degrees, about 33.5 degrees, about 34 degrees, about 34.5 degrees, about 35 degrees, about 35.5 degrees, about 36 degrees, about 36.5 degrees, about 37 degrees, about 37.5 degrees, about 38 degrees, about 38.5 degrees, about 39 degrees, about 39.5 degrees, about 40 degrees, or any value or range between those values.

With reference to Figure 24, the centreline CG of each grip 601 with respect to the centre C of the humidifier liquid chamber defines a centreline offset CO which creates a moment M due to the weight of the humidifier liquid chamber. Minimising this offset results in a reduction in the moment but increases the required diameter of the user's grasp which could limit usability. Therefore, the described configuration provides an appropriate balance between grasping diameter and centreline offset CO.

Each grip 601 is at a grip distance GD from the centre C of the humidifier liquid chamber.

This grip distance is between about 50 mm and about 70 mm. In some configurations, the grip distance GD is between about 55 mm and about 65 mm. In some configurations, the grip distance GD is about 55 mm, about 56 mm, about 57 mm, about 58 mm, about 59 mm, about 60 mm, about 61 mm, about 62 mm, about 63 mm, about 64 mm, about 65 mm, or any value or range between those values.

The grips 601 are located on opposing sides of the humidifier liquid chamber 151.

The grips 601 extend downwardly from the roof 156 at the top of the humidifier liquid chamber 151. The upper portions of the grips 601 terminate at the roof 156.

In the form shown, the grips 601 extend part way down from the roof 156 of the humidifier liquid chamber, so as to not reach the base 154 of the humidifier liquid chamber. With that configuration, the lower portions of the grips 601 terminate above the base 154 and base flange 155, and there is an uninterrupted part of the side wall(s) 153 beneath the grips 601. This minimises the chance of the user's hand contacting the breathing assistance apparatus base unit 50 during connection or contacting the base 154 or base flange 155 of the humidifier liquid chamber.

The grips 601 are vertically elongate, so that their vertical dimensions are larger than their forward-rearward dimensions.

In the form shown, the grips 601 comprise inward recesses 603. In the form shown, the recesses 603 are scalloped.

The contact patch created by the user's thumb or fingers within a recess of the grip is dependent on the depth of the recess, which is effectively governed by the radii of the circles which defines this recess.

Referring to Figure 25, significant dimensions for the grips 601 include one or more of the radius GR of the recess circle, the diameter of the 'construction circle' CC, and the angle α between centre of recess circle and the centre C of the humidifier liquid chamber. Advantageously, the dimensions are identical for each recess for the grip feature to be ambidextrous.

Increasing the radius GR of the recess circle and the diameter of the construction circle CC leads to a shallower recess which may be functionally less useful. Reducing the radius GR of the recess circle could define a recess too narrow for a user's fingers. Changes in the diameter of the construction circle CC are somewhat dependent on the radius GR of the recess circle and vice versa, a great mismatch could lead to a highly subtractive feature which would have a knock-on effect on the liquid fill level within the humidifier liquid chamber. If the grip radius GR was too large, the maximum liquid fill level MFL would be at a different height, which could affect the required height of the lower edge 403 of the baffle 401.

The grip recesses 603 have a radius GR of between about 10 mm and about 20 mm.

In some configurations, the grip recesses 603 have a radius GR of about 10 mm, about 11 mm, about 12 mm, about 13 mm, about 14 mm, about 15 mm, about 16 mm, about 17 mm, about 18 mm, about 19 mm, about 20 mm, or any value or range between those values.

In some configurations, the diameter of the construction circle is about 110 mm to about 122 mm, about 110 mm, about 111 mm, about 112 mm, about 113 mm, about 114 mm, about 115 mm, about 116 mm, about 117 mm, about 118 mm, about 119 mm, about 120 mm, about 121 mm, about 122 mm, or any value or range between those values.

Decreasing the angle α between centre of recess the circle and the centre C of the humidifier liquid chamber leads to an increase in the required grasping diameter. Suitable ranges for angle α are outlined above.

In some configurations, the grips 601 have substantially smooth grip surfaces. In some configurations, the grips 601 have textured grip surfaces.

The inlet and outlet ports 157, 159 of the humidifier liquid chamber are located at a front of the humidifier liquid chamber. The grips 601 are located in sides of the humidifier liquid chamber, rearward of the plane IP.

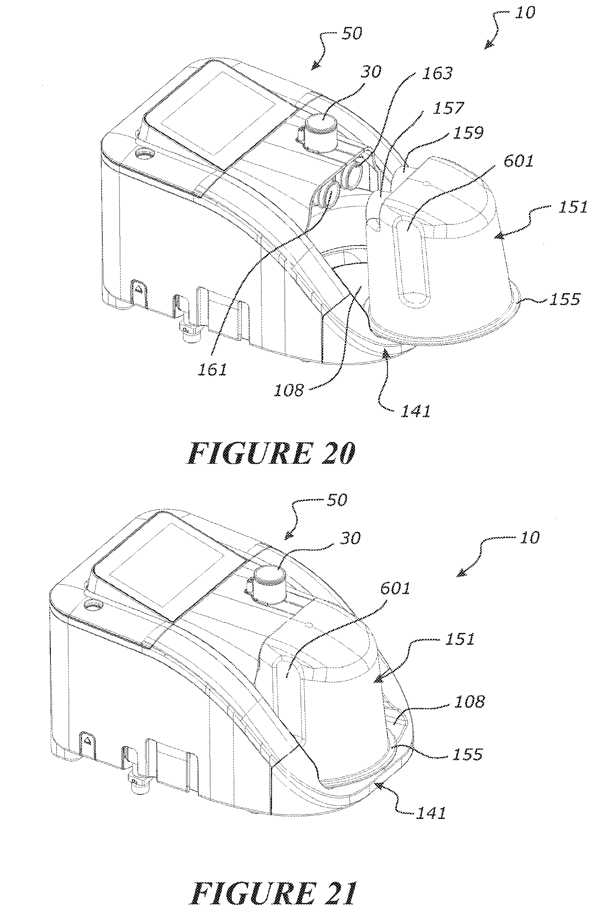

The humidifier liquid chamber 151 is configured to engage with the liquid chamber recess 108 of the breathing assistance apparatus by sliding the humidifier liquid chamber substantially linearly into the recess, from the front of the liquid chamber bay (Figure 20) to the rear of the liquid chamber bay (Figure 21). Alternatively, this motion could be in a lateral direction in a normal orientation of the breathing assistance apparatus.

The grips 601 are together an ambidextrous grip feature and enable thumb abducted large diameter grasp. It is expected that a user will rest a thumb within one recess and one or more fingers in the opposite recess to grasp the humidifier liquid chamber 151. Either hand could be used; there is no bias or difference in the geometry of either recess. The grip feature is advantageously a subtractive feature to fit within the breathing assistance apparatus base unit 50, whereas an additive feature, in the form of a handle or similar, might interfere with the breathing assistance apparatus or increase the likelihood of the user's hand contacting the breathing assistance apparatus base unit 50.

This is also preferable to just having a surface texture which might have a lesser effect on improving effective grasp strength, though a surface texture could be applied to either or both recesses. The grip feature encourages the user to grasp the humidifier liquid chamber in a way that makes port alignment and connection easier.

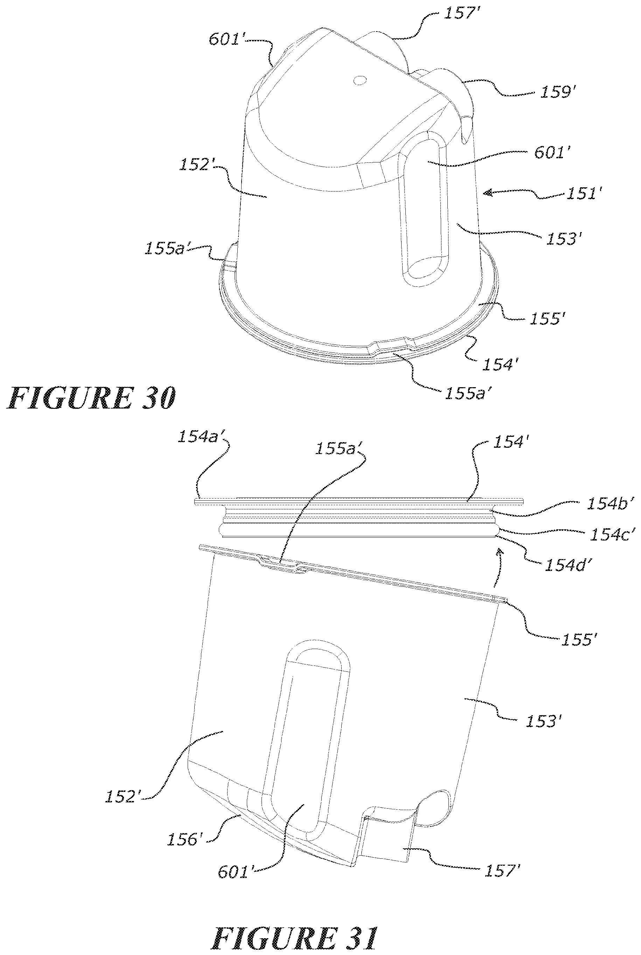

The humidifier liquid chamber may have a removable base. Figures 30 and 31 show an alternative configuration of the humidifier liquid chamber 151' with a removable heat conductive base 154'. Unless otherwise described below, the features, options, and functionality of the alternative configuration humidifier liquid chamber 151' are the same as for humidifier liquid chamber 151, and like reference numbers indicate like parts with the addition of a prime (').

The base 154' of the humidifier liquid chamber 151' has an annular outwardly extending flange 154a' that is substantially the same size as, or slightly larger or smaller than, the lower flange 155' of the body 152' of the humidifier liquid chamber. A cylindrical body 154b' extends upwardly from the flange 154a' (when the humidifier liquid chamber 151' is in an upright configuration).

A seal 154c', which may be any suitable type of seal such as an O-ring seal or wiper seal for example, is received in a seal recess 154d' of the cylindrical body 154b'. The seal 154c' engages against an inner surface of the body 152' of the humidifier liquid chamber to form a seal therebetween and to hold the base 154' in engagement with the body 152' of the humidifier liquid chamber.

At least one base removal feature 155a' is provided between the lower flange 155' of the body of the humidifier liquid chamber and the outwardly extending flange 154a'.

In the form shown, the base removal feature comprises a slot 155a' that is provided by an upwardly stepped region of the lower flange 155' of the body of the humidifier liquid chamber. Alternatively, the slot 155a' could be provided by a downwardly stepped region of the outwardly extending flange 154a' of the base 154', or by both an upwardly stepped region of the lower flange 155' of the body of the humidifier liquid chamber and a downwardly stepped region of the outwardly extending flange 154a' of the base 154'.

The slot is configured such that part of a suitable implement such as the blade of a flat-blade screwdriver, the blade of a flat-blade knife, or part of a coin, can be inserted into the slot and turned or levered to prise the base 154' off the body 152' of the humidifier liquid chamber.

In the form shown, the humidifier liquid chamber 151' has two spaced slots 155a'. In alternative configurations, the humidifier liquid chamber 151' may have one, two, three, or more spaced slots 155a'. The slots 155a' may be angularly spaced.

An additional or alternative removal feature 155a' comprises a difference in the periphery of the lower flange 155' of the body and flange 154a' of the base. Flange 155' and flange 154a' may not correspond exactly in shape such that the lower flange 155' could have a recessed edge along at least one portion of its periphery. This provides another way of removing the base 154 from the body of the humidifier liquid chamber; e.g., with the humidifier liquid chamber 151 turned upside down, a user can use his thumbs to lever the base off the body by pressing against the flange 154a'.

The base 154' may be removed to aid cleaning of the humidifier liquid chamber.

Following cleaning, the base 154' can press-fit back onto the lower edge of the body with the seal 154c' engaging against the inner surface of the body, by moving the body 152' of the humidifier liquid chamber in the direction of the arrow in Figure 31 toward the base 154', or by moving the base 154' of the humidifier liquid chamber toward the body 152' in a direction opposite the arrow in Figure 31.

As outlined above, the features that are shown in the figures and described above for the humidifier liquid chamber 151 can be implemented in a humidifier liquid chamber manifold 1701 for use with an alternative humidifier liquid chamber 1151, as shown in Figures 26 to 29. The features will not be described in full detail again below.

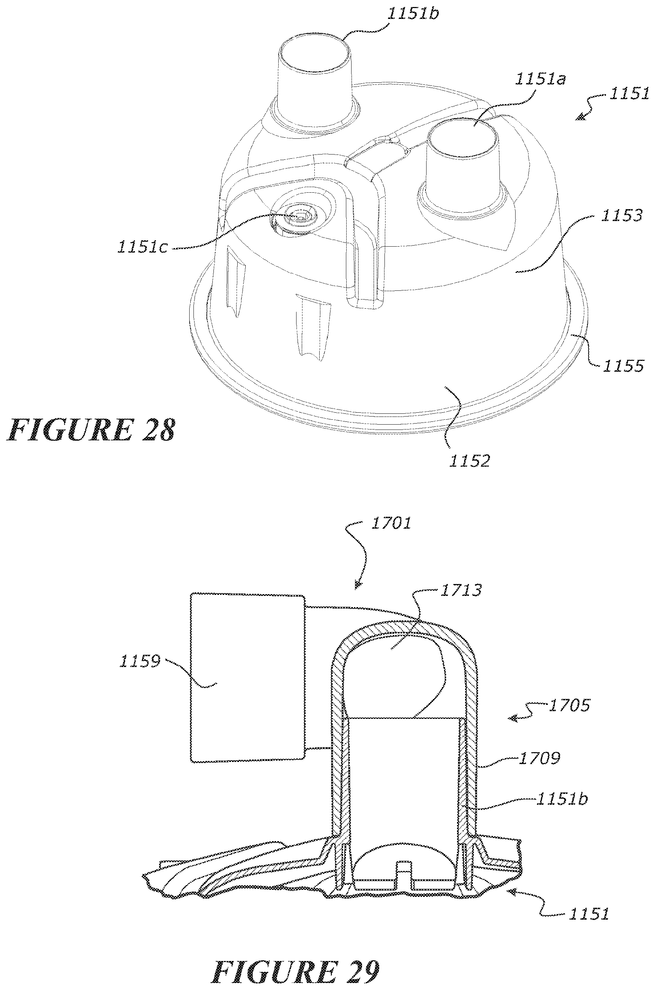

In the form shown, the humidifier liquid chamber 1151 has a lower height than humidifier liquid chamber 151. Upstanding connection ports 1151a, 1151b on the humidifier liquid chamber 1151 (one of which 1151b is shown in Figure 29, but both of which will typically have the same configuration) are spaced further apart than the port separation distance PSD of the ports 157, 159 of the humidifier liquid chamber 151.

The humidifier liquid chamber 1151 may have a removable base as described above.

The humidifier liquid chamber manifold 1701 comprises a base unit connection portion 1703 and a manifold-to-chamber connection portion 1705.

The base unit connection portion 1703 comprises the first base unit connection port 1157 and the second base unit connection port 1159 for connecting to the breathing assistance apparatus base unit 50, the base unit connection ports 1157, 1159 defining respective axes A1, A2.

The first base unit connection port 1157 is configured to connect to a complementary chamber connection port 161 of the breathing assistance apparatus base unit 50. The second base unit connection port 1159 is configured to connect to a complementary chamber connection port 163 of the breathing assistance apparatus base unit 50.

The base unit connection ports 1157, 1159 are substantially parallel to each other and are separated by a port separation distance PSD between the axes A1, A2.

The humidifier liquid chamber connection portion 1705 comprises first and second manifold-to-chamber connection ports 1707, 1709 for connecting to respective ports on the humidifier liquid chamber 1151.

The first and second manifold-to-chamber connection ports 1707, 1709 are configured to connect to complementary connection ports 1151a, 1151b on the humidifier liquid chamber 1151.

The first and second manifold-to-chamber connection ports 1707, 1709 are spaced further apart than the base unit connection ports 1157, 1159, due to the relatively wide spacing of the complementary connection ports 1151a, 1151b of the humidifier liquid chamber. However, that spacing can vary depending on the spacing of the complementary connection ports 1151a, 1151b of the humidifier liquid chamber. For example, the first and second manifold-to-chamber connection ports 1707, 1709 can be spaced less than or equal to the port separation distance PSD of the base unit connection ports 1157, 1159.

The first manifold-to-chamber connection port 1707 is in fluid communication with the first base unit connection port 1157. The second manifold-to-chamber connection port 1709 is in fluid communication with the second base unit connection port 1159. A first conduit 1711 extends between and fluidly connects the first manifold-to-chamber connection port 1707 and the first base unit connection port 1157. A second conduit 1713 extends between and fluidly connects the second manifold-to-chamber connection port 1709 and the second base unit connection port 1159.

An elbow can be formed in the first conduit 1711 and/or the second conduit 1713 due to a change from a substantially vertical to a substantially horizontal orientation, or vice versa.

At least one of, and optionally both of, the base unit connection ports 1157, 1159 has a sealing depth SD defined by a portion of the port 1157, 1159 that is configured to overlap with a complementary connection port 161, 163 of the breathing assistance apparatus 10.

The at least one of the base unit connection ports 1157, 1159 is configured to interact with a seal 173 of at least one of the chamber connection ports 161, 163.

In the form shown, the connection ports 161, 163 are male connection members and the base unit connection ports 1157, 1159 are female connection members. Alternatively, the connection ports 161, 163 could be female connection member and the base unit connection ports 1157, 1159 could be male connection members.

A ratio of the sealing depth SD to port separation distance PSD is more than about 0.25, more than about 0.25 and up to about 0.7, between about 0.3 and about 0.7, between about 0.3 and about 0.6, between about 0.4 and 0.5, between about 0.45 and 0.5, or about 0.475. The ratio of sealing depth SD to port separation distance PSD may be about 0.25, about 0.275, about 0.3, about 0.325, about 0.35, about 0.375, about 0.4, about 0.425, about 0.45, about 0.475, about 0.5, about 0.525, about 0.55, about 0.575, about 0.6, about 0.625, about 0.65, about 0.675, about 0.7, or any value or range between those values.

The sealing depth SD is more than about 10 mm, more than about 10 mm and up to about 16mm, more than about 12 mm, or more than about 12 mm and up to about 16 mm. The sealing depth SD may be about 10 mm, about 11 mm, about 12 mm, about 13 mm, about 14 mm, about 15 mm, about 16 mm, or any value or range between those values.

The port separation distance PSD is between about 35 mm and about 45 mm, between about 38 mm and about 42 mm, or is about 40 mm +/- 0.6mm. The port separation distance may be about 35 mm, about 36 mm, about 37 mm, about 38 mm, about 39 mm, about 40 mm, about 41 mm, about 42 mm, or any value or range between those values.

The at least one of the base unit connection ports 1157, 1159 is configured to seal with at least two seals 175 on the complementary chamber connection port 161, 163.

In some configurations, both of the base unit connection ports 1157, 1159 are configured to overlap with the complementary chamber connection ports 161, 163.

In some configurations, both of the base unit connection ports 1157, 1159 are configured to interact with a seal 173 of the chamber connection ports 161, 163.

In some configurations, each of the base unit connection ports 1157, 1159 is configured to seal with at least two seals 175 on the complementary chamber connection ports 161, 163.

The overlapping length of the base unit connection ports 1157, 1159 and the chamber connection ports 161, 163 is sufficient to provide improved protection against liquid ingress.

The base unit connection ports 1157, 1159 each have an exterior face 1158, 1160. The exterior faces 1158, 1160 are front faces of the base unit connection ports 1157, 1159 and are at an end of the base unit connection ports that are distal from the humidifier liquid chamber connection portion 1705.

The exterior faces 1158, 1160 are substantially planar and are substantially perpendicular to the axes A1, A2 of the base unit connection ports.

The manifold may comprise an auxiliary port (not shown) that is in communication with an interior of the manifold. The manifold may receive a sensor or may receive a gas source to act as a nebuliser.

The manifold comprises a conduit clip 1715 to engage with a liquid conduit for delivering liquid to a humidifier liquid chamber 1151. The humidifier liquid chamber 1151 comprises a liquid refilling aperture 1151c to receive liquid from the liquid conduit. This enables the liquid level in the humidifier liquid chamber to be maintained without disconnecting the humidifier liquid chamber 1151 from the breathing assistance apparatus base unit 50.

The humidifier liquid chamber manifold 1701 may be integrally formed as part of the humidifier liquid chamber 1151. For example, the humidifier liquid chamber manifold 1701 and humidifier liquid chamber 1151 may be integrally formed by an injection moulding process.

Alternatively, the humidifier liquid chamber manifold 1701 may be formed separately from the humidifier liquid chamber 1151, and may be coupled together with the humidifier liquid chamber. For example, the first and second manifold-to-chamber connection ports 1707, 1709 may couple (either removably or permanently) to the complementary inlet and outlet ports 1151a, 1151b on the humidifier liquid chamber. Such a configuration may enable the humidifier liquid chamber manifold 1701 to be retrofitted to an existing humidifier liquid chamber 1151, to enable the humidifier liquid chamber 1151 to be used with the breathing assistance apparatus 10.

In the form shown, the first and second manifold-to-chamber connection ports 1707, 1709 are tapered so that their inner dimensions are larger at the lower ends of the ports than higher up the ports, when the humidifier liquid chamber manifold 1701 is oriented so that the first and second manifold-to-chamber connection ports 1707, 1709 are extending downwardly. The complementary inlet and outlet ports 1151a, 1151b are tapered so that their outer dimensions are smaller at the upper ends of the ports than lower down the ports, when the humidifier liquid chamber 1151 is resting on its base and the inlet and outlet ports 1151a, 1151b are extending upwardly. This enables the ports to engage together in a taper lock, minimising the likelihood of the manifold accidentally being removed from the humidifier liquid chamber 1151.

Other types of connection could be used, such as a non-tapered friction fit or fasteners for example.

In the form shown, the axes A1, A2 of the first and second base unit connection ports 1157, 1159 are configured to be in a substantially horizontal orientation when the humidifier liquid chamber manifold 1701 is in use. In the form shown, the axes A3, A4 of the first and second manifold-to-chamber connection ports 1707, 1709 are configured to be in a substantially vertical orientation when the humidifier liquid chamber manifold 1701 is in use. This enables the humidifier liquid chamber 1151 with substantially vertical ports 1151a, 1151b to be used with a breathing assistance apparatus 10 that has substantially horizontal ports 161, 163.

It will be appreciated that the relative angles between the ports 1157, 1159 and 1707, 1709 can be varied depending on the configurations of the humidifier liquid chamber ports 1151a, 1151b and the breathing assistance apparatus ports 161, 163.

The manifold 1701 acts as an adapter to enable the humidifier liquid chamber 1151 (or any other suitable humidifier liquid chamber) to be used with the breathing assistance apparatus 10.

In the form shown, ports 1151a, 1151b are male connection members and ports 1707, 1709 are female connection members. Alternatively, ports 1151a, 1151b could be female connection members and ports 1707, 1709 could be male connection members

The humidifier liquid chamber manifold 1701 and humidifier liquid chamber 1151 may have any of the other features described or shown in relation to humidifier liquid chamber 151.

Although the present disclosure has been described in terms of certain embodiments, other embodiments apparent to those of ordinary skill in the art also are within the scope of this disclosure. Thus, various changes and modifications may be made without departing from the scope of the disclosure. For instance, various components may be repositioned as desired. Features from any of the described embodiments may be combined with each other and/or an apparatus may comprise one, more, or all of the features of the above described embodiments. Moreover, not all of the features, aspects and advantages are necessarily required to practice the present disclosure.

The various configurations described are exemplary configurations only. Any one or more features from any of the configurations may be used in combination with any one or more features from any of the other configurations.

The features are described with reference to a breathing assistance apparatus that can deliver heated and humidified gases to a patient or user. The apparatus may be suitable for treating chronic obstructive pulmonary disease (COPD). The apparatus may be configured to deliver gases to a patient interface at a high flow rate (high flow therapy), particularly nasal high flow therapy.

Alternatively, the features may be used with an apparatus for a different purpose. The apparatus may be a high flow therapy apparatus, or may be a low flow therapy apparatus. For example, the features may be provided in an apparatus for providing continuous positive airway pressure (CPAP), which may deliver gases (humidified or otherwise) at lower flow rates, or may be provided in a medical insufflation apparatus.

The features could be used in a standalone humidifier that has a housing, a recess 108 for receipt of the humidifier liquid chamber 151, and a heater plate 140, but that doesn't have a motor unit. The standalone humidifier may receive gases from an external source.

Reference to any prior art in this specification is not, and should not be taken as, an acknowledgement or any form of suggestion that the prior art forms part of the common general knowledge in the field of endeavour in any country in the world.
Where reference is used herein to directional terms such as 'up', 'down', 'forward', 'rearward', 'horizontal', 'vertical' etc, those terms refer to when the apparatus is in a typical in-use position and/or with reference to particular orientations shown in the figures, and are used to show and/or describe relative directions or orientations.
The invention is defined by the claims which follow.

## Claims

1. A humidifier liquid chamber (151) having a generally circular peripheral shape or a humidifier liquid chamber manifold (1701) comprising:
first and second base unit connection ports (157, 159) for connecting to a breathing assistance apparatus base unit, the base unit connection ports defining respective axes (A1, A2);
wherein the base unit connection ports (157, 159, 1157, 1159) are substantially parallel to each other and are separated by a port separation distance (PSD) between the axes (A1, A2), and the axes (A1, A2) of the first and second base unit connection ports (157, 159, 1157, 1159) are configured to be in a substantially horizontal orientation when the humidifier liquid chamber (151) or humidifier liquid chamber manifold (1701) is in use;
wherein at least one of the base unit connection ports (157, 159, 1157, 1159) has a sealing depth (SD) defined by a portion of the port that is configured to overlap with a complementary chamber connection port of the base unit, wherein a ratio of the sealing depth (SD) to port separation distance (PSD) is more than about 0.25 and up to about 0.7.

2. The humidifier liquid chamber (151) or humidifier liquid chamber manifold (1701) according to claim 1, wherein the ratio of the sealing depth (SD) to port separation distance (PSD) is between about 0.3 and about 0.7.

3. The humidifier liquid chamber (151) or humidifier liquid chamber manifold (1701) according to claim 1 or 2, wherein the sealing depth (SD) is more than about 12 mm and up to about 16 mm.

4. The humidifier liquid chamber (151) or humidifier liquid chamber manifold (1701) according to any one of claims 1 to 3, wherein said at least one of the base unit connection ports (157, 159, 1157, 1159) is configured to seal with at least two seals on the complementary chamber connection port.

5. The humidifier liquid chamber (151) or humidifier liquid chamber manifold (1701) according to any one of claims 1 to 4, wherein the base unit connection ports (157, 159, 1157, 1159) each have an exterior face (158, 160, 1158, 1160), and wherein the exterior faces (158, 160, 1158, 1160) are substantially planar and are substantially perpendicular to the axes (A1, A2).

6. The humidifier liquid chamber (151) or humidifier liquid chamber manifold (1701) according to any one of claims 1 to 5, wherein the port separation distance (PSD) is about 40 mm +/- 0.6mm.

7. The humidifier liquid chamber (151) according to any one of claims 1 to 6, comprising flow tubes (301) extending into the humidifier liquid chamber (151) from the first and second base unit connection ports (158, 160).

8. The humidifier liquid chamber (151) according to claim 7, wherein each flow tube (301) comprises an opening (309) at or adjacent an end of the flow tube (301) that is distal from the respective base unit connection port (158, 160), wherein the opening (309) is configured to direct flow and is spaced from a wall of the humidifier liquid chamber (151); optionally wherein the opening (309) in each flow tube (301) faces a roof (156) of the humidifier liquid chamber (151).

9. The humidifier liquid chamber (151) according to claim 8, comprising a baffle (401) in the interior of the humidifier liquid chamber (151) and between the openings (309) of the flow tubes (301), and wherein the baffle (401) extends downward from a roof (156) of the humidifier liquid chamber (151) and terminates above a maximum liquid fill level (MFL) of the humidifier liquid chamber (151).

10. The humidifier liquid chamber (151) according to claim 9, wherein a distance (D1) between a bottom edge (403) of the baffle (401) and the maximum liquid fill level (MFL) is between about 5 mm and about 15 mm.

11. The humidifier liquid chamber (151) according to any one of claims 1 to 10, comprising a step (501) in the humidifier liquid chamber (151).

12. The humidifier liquid chamber (151) according to claim 11, wherein a base (503) of the step (501) is located at least partly below the first and second base unit connection ports (158, 160).

13. The humidifier liquid chamber (151) according to claim 11 or 12 when dependent on claim 9 or 10, wherein the baffle (401) extends from a rear of the humidifier liquid chamber (151) towards a substantially vertical face (505) of the step (501), and terminates proximal to a central axis (CA) of the humidifier liquid chamber (151).

14. The humidifier liquid chamber (151) according to any one of claims 1 to 13, wherein the humidifier liquid chamber (151) has a substantially cylindrical shape; and optionally wherein the humidifier liquid chamber (151) tapers towards a centre (C) of the humidifier liquid chamber (151) from a base (154) of the humidifier liquid chamber to a top of the humidifier liquid chamber.

15. The humidifier liquid chamber (151) according to any one of claims 1 to 14, comprising a heat conductive base (154).

## Patentansprüche

1. Befeuchterflüssigkeitskammer (151) mit einer im Allgemeinen kreisförmigen Umfangsform oder ein Befeuchterflüssigkeitskammerverteiler (1701), umfassend:
erste und zweite Verbindungsanschlüsse (157, 159) der Basiseinheit zur Verbindung mit einer Basiseinheit eines Atemhilfegeräts, wobei die Verbindungsanschlüsse der Basiseinheiten jeweilige Achsen (A1, A2) definieren;
wobei die Verbindungsanschlüsse (157, 159, 1157, 1159) der Basiseinheit im Wesentlichen parallel zueinander sind und durch einen Anschlusstrennungsabstand (PSD) zwischen den Achsen (A1, A2) getrennt sind, und die Achsen (A1, A2) der ersten und zweiten Verbindungsanschlüsse (157, 159, 1157, 1159) der Basiseinheit konfiguriert sind, um in einer im Wesentlichen horizontalen Ausrichtung zu sein, wenn die Befeuchterflüssigkeitskammer (151) oder der Befeuchterflüssigkeitskammerverteiler (1701) in Verwendung ist;
wobei mindestens einer der Verbindungsanschlüsse (157, 159, 1157, 1159) der Basiseinheit eine Dichtungstiefe (SD) aufweist, die durch einen Abschnitt des Anschlusses definiert ist, der konfiguriert ist, um mit einem komplementären Kammerverbindungsanschluss der Basiseinheit zu überlappen, wobei ein Verhältnis der Dichtungstiefe (SD) zum Anschlusstrennungsabstand (PSD) mehr als etwa 0,25 und bis zu etwa 0,7 beträgt.

2. Befeuchterflüssigkeitskammer (151) oder Befeuchterflüssigkeitskammerverteiler (1701) nach Anspruch 1, wobei das Verhältnis von Dichtungstiefe (SD) zu Anschlusstrennungsabstand (PSD) zwischen etwa 0,3 und etwa 0,7 liegt.

3. Befeuchterflüssigkeitskammer (151) oder Befeuchterflüssigkeitskammerverteiler (1701) nach Anspruch 1 oder 2, wobei die Dichtungstiefe (SD) mehr als etwa 12 mm und bis zu etwa 16 mm beträgt.

4. Befeuchterflüssigkeitskammer (151) oder Befeuchterflüssigkeitskammerverteiler (1701) nach einem der Ansprüche 1 bis 3, wobei mindestens einer der Verbindungsanschlüsse (157, 159, 1157, 1159) der Basiseinheit konfiguriert ist, um mit mindestens zwei Dichtungen an dem komplementären Kammerverbindungsanschluss zu dichten.

5. Befeuchterflüssigkeitskammer (151) oder Befeuchterflüssigkeitskammerverteiler (1701) nach einem der Ansprüche 1 bis 4, wobei die Verbindungsanschlüsse (157, 159, 1157, 1159) der Basiseinheit jeweils eine äußere Fläche (158, 160, 1158, 1160) aufweisen, und wobei die äußeren Flächen (158, 160, 1158, 1160) im Wesentlichen eben sind und im Wesentlichen senkrecht zu den Achsen (A1, A2) stehen.

6. Befeuchterflüssigkeitskammer (151) oder Befeuchterflüssigkeitskammerverteiler (1701) nach einem der Ansprüche 1 bis 5, wobei der Anschlusstrennungsabstand (PSD) etwa 40 mm +/- 0,6 mm beträgt.

7. Befeuchterflüssigkeitskammer (151) nach einem der Ansprüche 1 bis 6, umfassend Flussrohre (301), die sich von den ersten und zweiten Verbindungsanschlüssen (158, 160) der Basiseinheit in die Befeuchterflüssigkeitskammer (151) erstrecken.

8. Befeuchterflüssigkeitskammer (151) nach Anspruch 7, wobei jedes Flussrohr (301) eine Öffnung (309) an oder neben einem Ende des Flussrohrs (301) umfasst, das von dem jeweiligen Verbindungsanschluss (158, 160) der Basiseinheit entfernt ist, wobei die Öffnung (309) konfiguriert ist, um den Fluss zu leiten und von einer Wand der Befeuchterflüssigkeitskammer (151) beabstandet ist; optional wobei die Öffnung (309) in jedem Flussrohr (301) einem Dach (156) der Befeuchterflüssigkeitskammer (151) gegenüberliegt.

9. Befeuchterflüssigkeitskammer (151) nach Anspruch 8, umfassend ein Ablenkblech (401) im Inneren der Befeuchterflüssigkeitskammer (151) und zwischen den Öffnungen (309) der Flussrohre (301), wobei sich das Ablenkblech (401) von einem Dach (156) der Befeuchterflüssigkeitskammer (151) nach unten erstreckt und über einem maximalen Flüssigkeitsfüllstand (MFL) der Befeuchterflüssigkeitskammer (151) endet.

10. Befeuchterflüssigkeitskammer (151) nach Anspruch 9, wobei ein Abstand (D1) zwischen einer unteren Kante (403) des Ablenkblechs (401) und dem maximalen Flüssigkeitsfüllstand (MFL) zwischen etwa 5 mm und etwa 15 mm beträgt.

11. Befeuchterflüssigkeitskammer (151) nach einem der Ansprüche 1 bis 10, umfassend einen Schritt (501) in der Befeuchterflüssigkeitskammer (151).

12. Befeuchterflüssigkeitskammer (151) nach Anspruch 11, wobei eine Basis (503) des Schritts (501) mindestens teilweise unter den ersten und zweiten Verbindungsanschlüssen (158, 160) der Basiseinheit angeordnet ist.

13. Befeuchterflüssigkeitskammer (151) nach Anspruch 11 oder 12, wenn sie von Anspruch 9 oder 10 abhängt, wobei sich das Ablenkblech (401) von einer Rückseite der Befeuchterflüssigkeitskammer (151) zu einer im Wesentlichen vertikalen Fläche (505) des Schritts (501) erstreckt und in der Nähe einer Mittelachse (CA) der Befeuchterflüssigkeitskammer (151) endet.

14. Befeuchterflüssigkeitskammer (151) nach einem der Ansprüche 1 bis 13, wobei die Befeuchterflüssigkeitskammer (151) eine im Wesentlichen zylindrische Form aufweist; und optional, wobei sich die Befeuchterflüssigkeitskammer (151) von einer Basis (154) der Befeuchterflüssigkeitskammer zu einem Zentrum (C) der Befeuchterflüssigkeitskammer (151) hin verjüngt.

15. Befeuchterflüssigkeitskammer (151) nach einem der Ansprüche 1 bis 14, umfassend eine wärmeleitfähige Basis (154).

## Revendications

1. Chambre de liquide d'humidificateur (151) ayant une forme périphérique généralement circulaire ou collecteur de chambre de liquide d'humidificateur (1701) comprenant :
de premier et second orifices de liaison d'unité de base (157, 159) permettant une liaison à une unité de base d'appareil d'assistance respiratoire, les orifices de liaison d'unité de base définissant des axes (A1, A2) respectifs ;
dans lequel les orifices de liaison d'unité de base (157, 159, 1157, 1159) sont sensiblement parallèles les uns aux autres et sont séparés par une distance de séparation d'orifice (PSD) entre les axes (A1, A2), et les axes (A1, A2) des premier et second orifices de liaison d'unité de base (157, 159, 1157, 1159) sont conçus pour être dans une orientation sensiblement horizontale lorsque la chambre de liquide d'humidificateur (151) ou le collecteur de chambre de liquide d'humidificateur (1701) est en cours d'utilisation ;
dans lequel au moins l'un des orifices de liaison d'unité de base (157, 159, 1157, 1159) a une profondeur d'étanchéité (SD) définie par une partie de l'orifice qui est conçue pour chevaucher un orifice de liaison de chambre complémentaire de l'unité de base, dans lequel un rapport entre la profondeur d'étanchéité (SD) et une distance de séparation d'orifice (PSD) est supérieur à environ 0,25 et jusqu'à environ 0,7.

2. Chambre de liquide d'humidificateur (151) ou collecteur de chambre de liquide d'humidificateur (1701) selon la revendication 1, dans lequel le rapport entre la profondeur d'étanchéité (SD) et une distance de séparation d'orifice (PSD) est compris entre environ 0,3 et environ 0,7.

3. Chambre de liquide d'humidificateur (151) ou collecteur de chambre de liquide d'humidificateur (1701) selon la revendication 1 ou 2, dans lequel la profondeur d'étanchéité (SD) est supérieure à environ 12 mm et jusqu'à environ 16 mm.

4. Chambre de liquide d'humidificateur (151) ou collecteur de chambre de liquide d'humidificateur (1701) selon l'une quelconque des revendications 1 à 3, dans lequel ledit au moins un des orifices de liaison d'unité de base (157, 159, 1157, 1159) est conçu pour assurer l'étanchéité avec au moins deux joints sur l'orifice de liaison de chambre complémentaire.

5. Chambre de liquide d'humidificateur (151) ou collecteur de chambre de liquide d'humidificateur (1701) selon l'une quelconque des revendications 1 à 4, dans lequel les orifices de liaison d'unité de base (157, 159, 1157, 1159) ont chacun une face extérieure (158, 160, 1158, 1160), et dans lequel les faces extérieures (158, 160, 1158, 1160) sont sensiblement planes et sont sensiblement perpendiculaires aux axes (A1, A2).

6. Chambre de liquide d'humidificateur (151) ou collecteur de chambre de liquide d'humidificateur (1701) selon l'une quelconque des revendications 1 à 5, dans lequel la distance de séparation d'orifice (PSD) est d'environ 40 mm +/- 0,6 mm.

7. Chambre de liquide d'humidificateur (151) selon l'une quelconque des revendications 1 à 6, comprenant des tubes d'écoulement (301) s'étendant dans la chambre de liquide d'humidificateur (151) à partir des premier et second orifices de liaison d'unité de base (158, 160).

8. Chambre de liquide d'humidificateur (151) selon la revendication 7, dans laquelle chaque tube d'écoulement (301) comprend une ouverture (309) au niveau d'une extrémité du tube d'écoulement (301) ou à proximité de celle-ci qui est distale par rapport à l'orifice de liaison d'unité de base (158, 160) respectif, dans laquelle l'ouverture (309) est conçue pour diriger un écoulement et est espacée d'une paroi de la chambre de liquide d'humidificateur (151) ; éventuellement, dans laquelle l'ouverture (309) dans chaque tube d'écoulement (301) fait face à un toit (156) de la chambre de liquide d'humidificateur (151).

9. Chambre de liquide d'humidificateur (151) selon la revendication 8, comprenant un déflecteur (401) à l'intérieur de la chambre de liquide d'humidificateur (151) et entre les ouvertures (309) des tubes d'écoulement (301), et dans laquelle le déflecteur (401) s'étend vers le bas à partir d'un toit (156) de la chambre de liquide d'humidificateur (151) et se termine au-dessus d'un niveau de remplissage de liquide maximal (MFL) de la chambre de liquide d'humidificateur (151).

10. Chambre de liquide d'humidificateur (151) selon la revendication 9, dans laquelle une distance (D1) entre un bord inférieur (403) du déflecteur (401) et le niveau de remplissage de liquide maximal (MFL) est comprise entre environ 5 mm et environ 15 mm.

11. Chambre de liquide d'humidificateur (151) selon l'une quelconque des revendications 1 à 10, comprenant une marche (501) dans la chambre de liquide d'humidificateur (151).

12. Chambre de liquide d'humidificateur (151) selon la revendication 11, dans laquelle une base (503) de la marche (501) est située au moins partiellement sous les premier et second orifices de liaison d'unité de base (158, 160).

13. Chambre de liquide d'humidificateur (151) selon la revendication 11 ou 12 lorsqu'elle dépend de la revendication 9 ou 10, dans laquelle le déflecteur (401) s'étend d'un arrière de la chambre de liquide d'humidificateur (151) vers une face sensiblement verticale (505) de la marche (501), et se termine à proximité d'un axe central (CA) de la chambre de liquide d'humidificateur (151).

14. Chambre de liquide d'humidificateur (151) selon l'une quelconque des revendications 1 à 13, dans laquelle la chambre de liquide d'humidificateur (151) a une forme sensiblement cylindrique ; et éventuellement dans laquelle la chambre de liquide d'humidificateur (151) se rétrécit vers un centre (C) de la chambre de liquide d'humidificateur (151) depuis une base (154) de la chambre de liquide d'humidificateur vers un sommet de la chambre de liquide d'humidificateur.

15. Chambre de liquide d'humidificateur (151) selon l'une quelconque des revendications 1 à 14, comprenant une base conductrice de chaleur (154).
